# EUROPEAN PATENT APPLICATION

(11) **EP 2 246 422 A1**
(43) Date of publication of application: **03.11.2010**
(21) Application number: 08871274.0
(22) Date of filing: 04.12.2008
(51) Int. Cl.: C12N 15/09, A61K 31/7105, A61P 3/04, A61P 3/06, A61P 3/10, A61P 9/00, A61P 9/10, A61P 9/12, A61P 35/00, C07H 21/02

(54) **POLYNUCLEOTIDE OR ANALOGUE THEREOF, AND GENE EXPRESSION REGULATION METHOD USING THE POLYNUCLEOTIDE OR THE ANALOGUE THEREOF**

(30) Priority: 24.01.2008 JP 2008013698
(71) Applicant: National Institute of Advanced Industrial Science And Technology, Tokyo 100-8921 (JP)
(72) Inventor: NATSUME, Toru, Tokyo 135-0064 (JP); ADACHI, Shungo, Tokyo 135-0064 (JP); IEMURA, Shunichiro, Tokyo 135-0064 (JP)
(74) Representative: TBK-Patent
(86) International application number: PCT/JP2008/072088
(87) International publication number: WO 2009/093384

(57) **Abstract**

[Objective]

To be provided is a gene expression regulation method of regulating expression of a target gene and regulating expression of a target gene product both positively and negatively.

[Means to Achieve Objectives]

Provided is a polynucleotide or polynucleotide analogue, comprising a nucleotide sequence complementary to at least part of a cis element of mRNA and a nucleotide sequence complementary to part of the 5'- and/or 3'- untranslated region of the cis element, wherein the polynucleotide or polynucleotide analogue inhibits binding of the cis element-binding factor to the cis element, by binding to at least part of the cis element specifically, based on the sequence specificity of the nucleotide sequence complementary to the untranslated region. The polynucleotide or polynucleotide analogue stabilizes the mRNA and accelerates translation thereof to the gene product or destabilizes it and inhibits translation thereof to the gene product selectively, by inhibiting binding of the cis element-binding factor to the cis element.

### [Sequence table]

SEQUENCE LISTING

<110> National Institute of Advanced Industrial Science and Technology

<120> Polynucleotide and its derivatives

<130> FPCT79

<160> 14

<170> PatentIn version 3.1

<210> 1
<211> 11
<212> DNA
<213> Artificial

<220>
<223> RNA/LNA hybrid sequence of LNA1

<400> 1
gaataaatat a                                                    11

<210> 2
<211> 19
<212> DNA
<213> Artificial

<220>
<223> RNA/LNA hybrid sequence of LNA2

<400> 2
tcccagatga ataaatata                                            19

<210> 3
<211> 11
<212> DNA
<213> Artificial

<220>
<223> RNA/LNA hybrid sequence of LNA3

<400> 3
agatgaataa a                                                    11

<210> 4
<211> 5175
<212> DNA
<213> Homo sapiens

<400> 4
gccccgagtg caatcgcggg aagccagggt ttccagctag gacacagcag gtcgtgatcc 60

gggtcgggac actgcctggc agaggctgcg agcatggggc cctggggctg gaaattgcgc 120

tggaccgtcg ccttgctcct cgccgcggcg gggactgcag tgggcgacag atgtgaaaga 180

aacgagttcc agtgccaaga cgggaaatgc atctcctaca agtgggtctg cgatggcagc 240

gctgagtgcc aggatggctc tgatgagtcc caggagacgt gcttgtctgt cacctgcaaa 300

tccggggact tcagctgtgg gggccgtgtc aaccgctgca ttcctcagtt ctggaggtgc 360

gatggccaag tggactgcga caacggctca gacgagcaag gctgtccccc caagacgtgc 420

tcccaggacg agtttcgctg ccacgatggg aagtgcatct ctcggcagtt cgtctgtgac 480

tcagaccggg actgcttgga cggctcagac gaggcctcct gcccggtgct cacctgtggt 540

cccgccagct tccagtgcaa cagctccacc tgcatccccc agctgtgggc ctgcgacaac 600

gaccccgact gcgaagatgg ctcggatgag tggccgcagc gctgtagggg tctttacgtg 660

ttccaagggg acagtagccc ctgctcggcc ttcgagttcc actgcctaag tggcgagtgc 720

atccactcca gctggcgctg tgatggtggc cccgactgca aggacaaatc tgacgaggaa 780

aactgcgctg tggccacctg tcgccctgac gaattccagt gctctgatgg aaactgcatc 840

catggcagcc ggcagtgtga ccgggaatat gactgcaagg acatgagcga tgaagttggc 900

tgcgttaatg tgacactctg cgagggaccc aacaagttca agtgtcacag cggcgaatgc 960

atcaccctgg acaaagtctg caacatggct agagactgcc gggactggtc agatgaaccc 1020

atcaaagagt gcgggaccaa cgaatgcttg gacaacaacg gcggctgttc ccacgtctgc 1080

aatgacctta agatcggcta cgagtgcctg tgccccgacg gcttccagct ggtggcccag 1140

cgaagatgcg aagatatcga tgagtgtcag gatcccgaca cctgcagcca gctctgcgtg 1200

aacctggagg gtggctacaa gtgccagtgt gaggaaggct tccagctgga cccccacacg 1260

aaggcctgca aggctgtggg ctccatcgcc tacctcttct tcaccaaccg gcacgaggtc 1320

aggaagatga cgctggaccg gagcgagtac accagcctca tccccaacct gaggaacgtg 1380

gtcgctctgg acacggaggt ggccagcaat agaatctact ggtctgacct gtcccagaga 1440

atgatctgca gcacccagct tgacagagcc cacggcgtct cttcctatga caccgtcatc 1500

agcagggaca tccaggcccc cgacgggctg gctgtggact ggatccacag caacatctac 1560

tggaccgact ctgtcctggg cactgtctct gttgcggata ccaagggcgt gaagaggaaa 1620

acgttattca gggagaacgg ctccaagcca agggccatcg tggtggatcc tgttcatggc 1680

ttcatgtact ggactgactg gggaactccc gccaagatca agaaaggggg cctgaatggt 1740

gtggacatct actcgctggt gactgaaaac attcagtggc ccaatggcat caccctagat 1800

ctcctcagtg gccgcctcta ctgggttgac tccaaacttc actccatctc aagcatcgat 1860

gtcaatgggg gcaaccggaa gaccatcttg gaggatgaaa agaggctggc ccaccccttc 1920

tccttggccg tctttgagga caaagtattt tggacagata tcatcaacga agccattttc 1980

agtgccaacc gcctcacagg ttccgatgtc aacttgttgg ctgaaaacct actgtcccca 2040

gaggatatgg tcctcttcca caacctcacc cagccaagag gagtgaactg gtgtgagagg 2100

accaccctga gcaatggcgg ctgccagtat ctgtgcctcc ctgccccgca gatcaacccc 2160

cactcgccca agtttacctg cgcctgcccg gacggcatgc tgctggccag ggacatgagg 2220

agctgcctca cagaggctga ggctgcagtg gccacccagg agacatccac cgtcaggcta 2280

aaggtcagct ccacagccgt aaggacacag cacacaacca cccggcctgt tcccgacacc 2340

tcccggctgc ctggggccac ccctgggctc accacggtgg agatagtgac aatgtctcac 2400

caagctctgg gcgacgttgc tggcagagga aatgagaaga agcccagtag cgtgagggct 2460

ctgtccattg tcctccccat cgtgctcctc gtcttccttt gcctgggggt cttccttcta 2520

tggaagaact ggcggcttaa gaacatcaac agcatcaact ttgacaaccc cgtctatcag 2580

aagaccacag aggatgaggt ccacatttgc cacaaccagg acggctacag ctacccctcg 2640

agacagatgg tcagtctgga ggatgacgtg gcgtgaacat ctgcctggag tcccgcccct 2700

gcccagaacc cttcctgaga cctcgccggc cttgttttat tcaaagacag agaagaccaa 2760

agcattgcct gccagagctt tgttttatat atttattcat ctgggaggca gaacaggctt 2820

cggacagtgc ccatgcaatg gcttgggttg ggattttggt ttcttccttt cctgtgaagg 2880

ataagagaaa caggcccggg gggaccagga tgacacctcc atttctctcc aggaagtttt 2940

gagtttctct ccaccgtgac acaatcctca aacatggaag atgaaagggc aggggatgtc 3000

aggcccagag aagcaagtgg ctttcaacac acaacagcag atggcaccaa cgggaccccc 3060

tggccctgcc tcatccacca atctctaagc caaaccccta aactcaggag tcaacgtgtt 3120

tacctcttct atgcaagcct tgctagacag ccaggttagc ctttgccctg tcacccccga 3180

atcatgaccc acccagtgtc tttcgaggtg ggtttgtacc ttccttaagc caggaaaggg 3240

attcatggcg tcggaaatga tctggctgaa tccgtggtgg caccgagacc aaactcattc 3300

accaaatgat gccacttccc agaggcagag cctgagtcac cggtcaccct taatatttat 3360

taagtgcctg agacacccgg ttaccttggc cgtgaggaca cgtggcctgc acccaggtgt 3420

ggctgtcagg acaccagcct ggtgcccatc ctcccgaccc ctacccactt ccattcccgt 3480

ggtctccttg cactttctca gttcagagtt gtacactgtg tacatttggc atttgtgtta 3540

ttattttgca ctgttttctg tcgtgtgtgt tgggatggga tcccaggcca gggaaagccc 3600

gtgtcaatga atgccgggga cagagagggg caggttgacc gggacttcaa agccgtgatc 3660

gtgaatatcg agaactgcca ttgtcgtctt tatgtccgcc cacctagtgc ttccacttct 3720

atgcaaatgc ctccaagcca ttcacttccc caatcttgtc gttgatgggt atgtgtttaa 3780

aacatgcacg gtgaggccgg gcgcagtggc ctcacgcctg taatcccagc actttgggag 3840

gccgaggcgg gtggatcatg aggtcaggag atcgagacca tcctggctaa caaggtgaaa 3900

ccccgtctct actaaaaata caaaaaatta gccgggcgcg gtggtgggca cctgtagtcc 3960

cagctactcg ggaggctgag gcaggagaat ggtgtgaacc cgggaagcgg agcttgcagt 4020

gagccgagat tgcgccactg cagtccgcag tctggcctgg gcgacagagc gagactccgt 4080

ctcaaaaaaa acaaaacaaa aaaaaaccat gcatggtgca tcagcagccc atggcctctg 4140

gccaggcatg gcgaggctga ggtgggagga tggtttgagc tcaggcattt gaggctgtcg 4200

tgagctatga ttatgccact gctttccagc ctgggcaaca tagtaagacc ccatctctta 4260

aaaaatgaat ttggccagac acaggtgcct cacgcctgta atcccagcac tttgggaggc 4320

tgagctggat cacttgagtt caggagttgg agaccaggcc tgagcaacaa agcgagatcc 4380

catctctaca aaaaccaaaa agttaaaaat cagctgggta tggtggcacg tgcctgtgat 4440

cccagctact tgggaggctg aggcaggagg atcgcctgag cccaggaggt ggaggttgca 4500

gtgagccatg atcgagccac tgcactccag cctgggcaac agatgaagac cctatttcag 4560

aaatacaact ataaaaaaaa taaataaatc ctccagtctg gatcgtttga cgggacttca 4620

ggttctttct gaaatcgccg tgttactgtt gcactgatgt ccggagagac agtgacagcc 4680

tccgtcagac tcccgcgtga agatgtcaca agggattggc aattgtcccc agggacaaaa 4740

cactgtgtcc cccccagtgc agggaaccgt gataagcctt tctggtttcg gagcacgtaa 4800

atgcgtccct gtacagatag tggggatttt ttgttatgtt tgcactttgt atattggttg 4860

aaactgttat cacttatata tatatataca cacatatata taaaatctat ttatttttgc 4920

aaaccctggt tgctgtattt gttcagtgac tattctcggg gccctgtgta gggggttatt 4980

gcctctgaaa tgcctcttct ttatgtacaa agattatttg cacgaactgg actgtgtgca 5040

acgctttttg ggagaatgat gtccccgttg tatgtatgag tggcttctgg gagatgggtg 5100

tcacttttta aaccactgta tagaaggttt ttgtagcctg aatgtcttac tgtgatcaat 5160

taaatttctt aaatg 5175

<210> 5
<211> 1793
<212> DNA
<213> Homo sapiens

<400> 5
cgcgtccgcc ccgcgagcac agagcctcgc ctttgccgat ccgccgcccg tccacacccg 60

ccgccagctc accatggatg atgatatcgc cgcgctcgtc gtcgacaacg gctccggcat 120

gtgcaaggcc ggcttcgcgg gcgacgatgc cccccgggcc gtcttcccct ccatcgtggg 180

gcgccccagg caccagggcg tgatggtggg catgggtcag aaggattcct atgtgggcga 240

cgaggcccag agcaagagag gcatcctcac cctgaagtac cccatcgagc acggcatcgt 300

caccaactgg gacgacatgg agaaaatctg gcaccacacc ttctacaatg agctgcgtgt 360

ggctcccgag gagcaccccg tgctgctgac cgaggccccc ctgaacccca aggccaaccg 420

cgagaagatg acccagatca tgtttgagac cttcaacacc ccagccatgt acgttgctat 480

ccaggctgtg ctatccctgt acgcctctgg ccgtaccact ggcatcgtga tggactccgg 540

tgacggggtc acccacactg tgcccatcta cgaggggtat gccctccccc atgccatcct 600

gcgtctggac ctggctggcc gggacctgac tgactacctc atgaagatcc tcaccgagcg 660

cggctacagc ttcaccacca cggccgagcg ggaaatcgtg cgtgacatta aggagaagct 720

gtgctacgtc gccctggact tcgagcaaga gatggccacg gctgcttcca gctcctccct 780

ggagaagagc tacgagctgc ctgacggcca ggtcatcacc attggcaatg agcggttccg 840

ctgccctgag gcactcttcc agccttcctt cctgggcatg gagtcctgtg gcatccacga 900

aactaccttc aactccatca tgaagtgtga cgtggacatc cgcaaagacc tgtacgccaa 960

cacagtgctg tctggcggca ccaccatgta ccctggcatt gccgacagga tgcagaagga 1020

gatcactgcc ctggcaccca gcacaatgaa gatcaagatc attgctcctc ctgagcgcaa 1080

gtactccgtg tggatcggcg gctccatcct ggcctcgctg tccaccttcc agcagatgtg 1140

gatcagcaag caggagtatg acgagtccgg cccctccatc gtccaccgca aatgcttcta 1200

ggcggactat gacttagttg cgttacaccc tttcttgaca aaacctaact tgcgcagaaa 1260

acaagatgag attggcatgg ctttatttgt tttttttgtt ttgttttggt tttttttttt 1320

tttttggctt gactcaggat ttaaaaactg gaacggtgaa ggtgacagca gtcggttgga 1380

gcgagcatcc cccaaagttc acaatgtggc cgaggacttt gattgcacat tgttgttttt 1440

ttaatagtca ttccaaatat gagatgcatt gttacaggaa gtcccttgcc atcctaaaag 1500

ccaccccact tctctctaag gagaatggcc cagtcctctc ccaagtccac acaggggagg 1560

tgatagcatt gctttcgtgt aaattatgta atgcaaaatt tttttaatct tcgccttaat    1620

acttttttat tttgttttat tttgaatgat gagccttcgt gccccccctt cccccttttt    1680

gtcccccaac ttgagatgta tgaaggcttt tggtctccct gggagtgggt ggaggcagcc    1740

agggcttacc tgtacactga cttgagacca gttgaataaa agtgcacacc tta    1793

<210>  6
<211>  11
<212>  DNA
<213>  Artificial

<220>
<223>  LNA sequence of LAN6

<400>  6
ctcccagatg a    11

<210>  7
<211>  11
<212>  DNA
<213>  Artificial

<220>
<223>  LNA sequence of LNA7

<400>  7
cccagatgaa t    11

<210>  8
<211>  11
<212>  DNA
<213>  Artificial

<220>
<223>  LNA sequence of LNA8

<400>  8
aaatatataa a    11

<210>  9
<211>  11
<212>  DNA

<213>  Artificial

<220>
<223>  LNA sequence of LNA9

<400>  9
atatataaaa c                                                          11


<210>  10
<211>  9
<212>  DNA
<213>  Artificial

<220>
<223>  LNA sequence of LNA10

<400>  10
agatgaata                                                             9


<210>  11
<211>  9
<212>  DNA
<213>  Artificial

<220>
<223>  LNA sequence of LNA11

<400>  11
atgaataaa                                                             9


<210>  12
<211>  8
<212>  DNA
<213>  Artificial

<220>
<223>  LNA sequence of LNA12

<400>  12
agatgaat                                                              8


<210>  13
<211>  8
<212>  DNA

<213>  Artificial

<220>
<223>  LNA sequence of LNA13

<400>  13
atgaataa                                                                8


<210>  14
<211>  8
<212>  DNA
<213>  Artificial

<220>
<223>  LNA sequence of LNA14

<400>  14
tgaataaa                                                                8

## Description

### Technical Field

The present invention relates to a polynucleotide or a polynucleotide analogue and others. More specifically, it relates to a polynucleotide or polynucleotide analogue having a nucleotide sequence complementary to at least part of cis element of mRNA that can regulate expression of the gene product, and to a gene expression regulation method by using the polynucleotide or the like.

### Background Art

Homeostasis of the body is maintained, as various genes involved in various biological reactions such as cell differentiation/growth, nerve transmission and immune reaction function, as their expression are regulated properly in response to external stimuli. The expression site, expression level, expression time and others of each gene should be regulated precisely and, if any gene is expressed in an excessively large or small amount over or below the normal regulatory range of the gene, the homeostasis is disturbed, leading the body into a pathological state such as cancer, chronic inflammation or autoimmune disease. Thus for prevention, alleviation, or treatment of diseases such as cancer, chronic inflammation and autoimmune disease, it is needed to regulate expression of the disease-related genes by inhibition or acceleration into a desired expression state.

Recently, in the field of regeneration therapy, attempts are made to differentiate stem cells into a desired cell type or alternatively to produce iPS cells with totipotency from cells in the differentiated state by regulation of gene expression in the cells. Proper regulation of gene expression is important not only for treatment and others of diseases, but also for artificial modification or optimization of the differentiation/proliferation potency and the immunocompetence of cells, as described above.

Regulation of intercellular gene expression is carried out in both phases of transcription and translation of mRNA, by modification of the amount of mRNA transcripted, the stability of the transcripted mRNA, the amount of the protein translated, and the stability of the protein itself. There have been so far developed various gene expression regulation methods that are targeted to each of these regulation phases.

An example thereof is "antisense method" of introducing a single-stranded RNA or DNA (antisense strand) complementary to a target gene mRNA (sense strand) into cells and selectively inhibiting translation of the sense strand into protein. The antisense method, which is higher in gene selectivity and acts on the target gene mRNA directly, is expected to be higher in effectiveness at low toxicity.

A gene expression regulation method that is also highly expected, similarly to the antisense method, is "RNA interference (RNAi)" method. The RNAi method is a method of introducing a double strand RNA (dsRNA) complementary to the target gene mRNA or low-molecular weight interfering RNAs (short interfering RNAs; siRNAs) generated by decomposition of the dsRNA by an enzyme called dicer in the RNase III family and decomposing the target gene mRNA specifically by the RISC (RNA-induced silencing complex) formed from the siRNAs and multiple proteins. Although the antisense method uses an antisense strand not present in nature, the RNAi method uses an existing intercellular gene expression regulating system and is thus expected to be higher in safety.

Along with discovery of the gene expression-regulating mechanisms via mRNA, such as those by the antisense and RNAi methods above, importance of expression regulation mechanism via regulation of the stability of mRNA and the translation amount thereof to protein is attracting intensive attention recently.

The stability and the translation amount of mRNA are regulated by a particular sequence of mRNA called "cis element" and a "cis element-binding factor" binding thereto.

The cis element, which is present in DNA and 5' and 3' untranslated regions of RNA, is defined as a region that is involved in regulation of expression of the gene coded in the DNA or RNA strand. The "cis element-binding factor" functions as a trans-acting factor that regulates expression of the gene positively or negatively by binding to the cis element of gene.

For example, promotors and enhancers have been well known as the cis elements present in DNA. Typical examples of the promotors include TATA box, CAT box, Sp1 and the like, and a universal transcription factor binds to the cis element as the cis element-binding factor, for regulation of gene expression at the transcription level.

On the other hand, the cis elements present in mRNA, which are involved in regulation of the stability of mRNA and the amount of the protein translated, are factors important in determining the expression level of the gene product (protein) coded by the mRNA.

A typical example of the cis element is "AU-rich element (ARE)". ARE is a nucleotide sequence having a length of about 10 to 150 bps higher in adenosine and uridine contents and present mainly in the 3' untranslated region (3'UTR) of mRNA. ARE was first found as a region in which the nucleotide sequence of "AUUUA" appears repeatedly in the 3'UTRs of cytokines and lymphokines. ARE is currently considered to be present in 5 to 8% of all genes and also in many genes involved in homeostasis (see Non patent Literature 1).

ARE-binding proteins bind to ARE as trans-acting factors and regulate the stability and the translation amount of mRNA positively or negatively (mainly negative) (see Non patent Literatures 2 and 3). In addition, microRNAs (miRNAs) also bind to ARE, performing similar regulation (see Non patent Literatures 4 and 5).

Hereinafter, a term "LNA (locked nucleic acid)" will be described, in relation to the present invention. Nucleosides in natural nucleic acids (DNAs and RNAs) have 2 kinds of conformations, i.e., N- and S-type conformations. Double strands formed between DNA-DNA, RNA-RNA, and DNA-RNA are not always thermodynamically stabilized, because of the "fluctuation" in conformation. 2',4'-LNA (synonymous with bridged nucleic acid; BNA) is a synthetic nucleic acid having a fixed n-type conformation in which the 2' and 4' sites of the riboses (saccharides) are crosslinked with "-O-CH₂-" bonds. There are approximately 10 kinds of LNAs developed so far, in addition to the 2',4'-LNA (see Patent Literatures 1 to 3). The oligonucleotide analogues prepared with LNA show no "fluctuation" in conformation that is found in oligonucleotides prepared from conventional natural nucleic acids, and thus, have an extremely large binding force to RNA and DNA and are also superior in sequence specificity. They are also superior in heat resistance and nuclease resistance, compared to conventional oligonucleotides.

[Patent Literature 1] Japanese Translation of PCT No. 2002-521310
[Patent Literature 2] Japanese Unexamined Patent Application Publication No. 10-304889
[Patent Literature 3] Japanese Unexamined Patent Application Publication No. 10-195098
[Non patent Literature 1] "ARED: human AU-rich element-containing mRNA database reveals an unexpectedly diverse functional repertoire of encoded proteins." Nucleic Acids Research, 2001, Vol. 29, No. 1, p. 246-254.
[Non patent Literature 2] "AU-rich elements and associated factors: are there unifying principles?" Nucleic Acids Research, 2005, Vol. 33, No. 22, p. 7138-7150
[Non patent Literature 3] "AU-rich element-mediated translational control: complexity and multiple activities of trans-activating factors." Biochemical Society Transactions, 2002, Vol. 30, part 6, p. 952-958
[Non patent Literature 4] "Involvement of microRNA in AU-rich element-mediated mRNA instability." Cell, 2005, Vol. 120, No. 5, p. 623-634
[Non patent Literature 5] "Mechanisms of translational control by the 3' UTR in development and differentiation." Seminars in Cell and Developmental Biology, 2005, Vol. 16, No. 1, p. 49-58

### Disclosure of the Invention

### Technical Problem

The gene expression regulation methods described above, such as antisense and RNAi methods, are methods of regulating expression of a target gene product negatively by inhibiting translation of a target gene mRNA or decomposing the mRNA, based on the sequence specificity of an antisense strand, a dsRNA or a siRNA. Thus, it is not possible by these methods to increase the expression level by regulating expression of the target gene product positively.

However, it is needed for prevention, alleviation, or treatment of a disease to bring expression of the disease-related gene properly into a desired expression state, not only by inhibiting gene expression, but also by accelerating gene expression as needed. It is also needed to induce expression of each gene at favorable timing, for artificial modification or optimization of the differentiation proliferation potency, the immunocompetence and others of cells.

For that reason, a major object of the present invention is to provide a new gene expression regulation method for regulation of expression of a target gene, by which expression of the target gene product can also be regulated positively.

### Solution to Problem

The present invention, which was made to solve the problems above, provides a polynucleotide or polynucleotide analogue, comprising a nucleotide sequence complementary to at least part of a cis element of mRNA and a nucleotide sequence complementary to part of the 5'-and/or 3'- untranslated region of the cis element, wherein the polynucleotide or polynucleotide analogue inhibits binding of the cis element-binding factor to the cis element, by binding to at least part of the cis element specifically, based on the sequence specificity of the nucleotide sequence complementary to the untranslated region.
The polynucleotide or polynucleotide analogue stabilizes the mRNA selectively and accelerates translation of the gene product or destabilizes the mRNA and inhibits translation of the gene product, by inhibiting binding of the cis element-binding factor to the cis element.
The cis element and the cis element-binding factor, the binding of which is inhibited by the polynucleotide or polynucleotide analogue, may be particularly an AU-rich element and an AU-rich element binding factor.
In the present invention, the polynucleotide analogue particularly preferably contains LNA.

The present invention also provides a gene product expression enhancer or inhibitor containing the polynucleotide and/or polynucleotide analogue or an expression vector that can express the polynucleotide and a pharmaceutical composition containing the gene product expression enhancer or inhibitor as the active ingredient.
The present invention also provides use of the polynucleotide and/or polynucleotide analogue or the expression vector that can express the polynucleotide, in particular the expression vector that can express the polynucleotide, in production of the target gene product expression enhancer or inhibitor.

The present invention further provides the polynucleotide or polynucleotide analogue above, wherein the mRNA is low-density lipoprotein receptor (LDLR) mRNA and binding of ZFP36L1 and/or ZFP36L2 to the AU-rich element is inhibited.
The polynucleotide and the polynucleotide analogue inhibits binding of the cis element-binding factor to ARE1 (2790 to 2797 bases from the 5' terminal side in the nucleotide sequence of the LDLR mRNA represented by SEQ ID No. 4).
The polynucleotide and polynucleotide analogue may have a nucleotide sequence represented by any one of SEQ ID Nos. 1 to 3 and 6 to 14 or a nucleotide sequence substantially equivalent to the nucleotide sequence.

The present invention also provides an LDLR expression enhancer, comprising one or more of the polynucleotides and/or polynucleotide analogues or one or more expression vectors that can express the polynucleotides. It also provides a pharmaceutical composition for prevention, alleviation, or treatment of hyperlipidemia, hyperlipidemia-related diseases or Alzheimer's disease, comprising the LDLR expression enhancer as the active ingredient. The hyperlipidemia-related disease may be one or more diseases selected from the group consisting of arteriosclerosis, hypertension, cerebral infarction, myocardial infarction, angina cordis, diabetes, adiposis and cancer.
The present invention also provides an expression vector that can express the polynucleotide.

It also provides a gene expression regulation method, comprising regulating expression of a target gene product by inhibiting binding of the cis element-binding factor to the cis element, using a polynucleotide and/or polynucleotide analogue, comprising a nucleotide sequence complementary to at least part of a cis element of mRNA and a nucleotide sequence complementary to part of the 5'- and/or 3'- untranslated region of the cis element.

Hereinafter, definitions of the terms used in the present invention will be described.

The term "polynucleotide" means a linear chain of nucleotides bound linearly to each other via phosphodiester bonds of the phosphoric acid groups. More specifically, it means a conventional nucleotide strand (DNA strand or RNA strand) of nucleic acids (DNAs or RNAs). The nucleotide is nucleosides bound to each other via phosphoric acid, and the nucleoside is a compound of a sugar (ribose) bound to a purine base such as adenine or guanine or a pyrimidine base such as thymine, cytosine or uracil. The bases, which have so-called complementarity, form base pairs by hydrogen bonding between adenine and thymine (uracil) and between guanine and cytosine. Thus, two polynucleotides (DNA/DNA, DNA/RNA, RNA/RNA) having complementary nucleotide sequences form a double strand, by binding thereof to each other at high affinity via hydrogen bonds between the base pairs. Polynucleotides consisting of several to dozens of nucleotides are particularly called oligonucleotides.

The term "polynucleotide analogue" means a polymer having a "nucleotide sequence-like structure" mimicking the nucleotide sequence of polynucleotide, i.e., the structure formed by binding of bases to a basic molecular chain of sugar chain. The "nucleotide sequence-like structure" of the polynucleotide analogue needs not have a structure in which the bases are aligned in sequence, and may have a structure in which compounds that can recognize and bind complimentarily to respective bases, adenine, guanine, cytosine and uracil are aligned in sequence, replacing the bases, as they are bound to a basic molecular chain. In the present invention the term "nucleotide sequence" includes the nucleotide sequence of a polynucleotide and also the nucleotide sequence-like structure of a "polynucleotide analogue". Also in the present invention, the concept of the term "length (base number)" includes the base number of a polynucleotide and also the number of compounds aligned in the basic molecular chain of the polynucleotide analogue.

The basic molecular chain of the polynucleotide analogue may not be formed via phosphodiester bonds of riboses and, for example, may contain riboses of which the structure is modified, such as the LNA described above or sugars other than riboses, or alternatively, these compounds may be bound to each other via chemical bonds other than phosphodiester bonds. The basic molecular chain is not particularly limited, if it is a molecular chain that can support the nucleotide sequence-like structure. The polynucleotide analogue, which has a structure in which the nucleotide sequence-like structure is supported by the basic molecular chain, can bind to a mRNA having a complementary nucleotide sequence at high affinity by the nucleotide sequence-like structure, forming a double strand.

The "cis elements" means regions that are present in the 5' and 3' untranslated regions of mRNA and are involved in regulation of expression of the gene coded by the mRNA strand, and typical examples thereof at least include AU-rich element, Histone mRNA 3' UTR stem loop element, Internal ribosome entry site (IRES), A2RE element, ZIPCODE element, Iron response element (IRE), Cytoplasmic polyadenylation (CPE), Nanos translational control, Amyloid precursor protein element (APP), Translational orientation element (TGE)/direct repeat element (DRE), Bruno element (BRE), 15-lipoxygenase differentiation control element (15-LOX-DICE), G-quartet element, Adh mRNA down-orientation element, Barley yellow dwarf virus, GLUT mRNA-stability control element, Msl-23 UTR control element, Msl-2 5 UTR control element, Ribosomal S12 mRNA translational control element, Selenocysteine insertion sequence type 1 (SECIS-1), Selenocysteine insertion sequence type 2 (SECIS-2), TNF-mRNA stability control element, Terminal oligopyrimidine tract (TOP), Vimentin mRNA 3 UTR control element and the like (see "Untranslated regions of mRNAs." Genome Biology. 2002, Vol. 3, No. 3, reviews 0004.1-0004.10). The "cis element-binding factor" is meant to include widely proteins and miRNAs that regulate expression of the gene positively or negatively by binding to the cis element.

The "AU-rich element (ARE)" means a nucleotide sequence of about 10 to 150 bps rich in adenosine and uridine contents that is involved in regulation of the stability and translation amount of mRNA and often present in 3'UTR of mRNA. The AREs are currently divided into the following three groups temporarily, and the three groups of AREs are at least included in the present invention (see the Non patent Literature 2): (1) a region (ARE I) containing several copies of "AUUUA pentamers" in uridine-rich sequence; (2) a region (ARE II) containing at least two repeated "UUAUUUA(U/A)(U/A) nonamers"; and (3) a uridine-rich region (ARE III) containing no "AUUUA pentamer".

The term "AU-rich element binding factor" is meant to include widely proteins and miRNAs that regulate expression of the gene coded by mRNA strand positively or negatively by binding to the ARE. Typical examples thereof at least include proteins such as AUF1, HuR, Hel-N1, HuD, TTP, BRF1, TIA-1, KSRP, GUG-BP2, Nucleotin, TINO, PAIP2, ZFP36L1 and ZFP36L2 (see Non patent Literature 2), miRNAs such as miR16 (see Non patent Literature 4), and the like.

### Advantageous Effects of Invention

The present invention provides a gene expression regulation method of regulating expression of a target gene, which can regulate expression of the target gene product also positively.

### Description of Embodiments

Hereinafter, favorable embodiments of the present invention will be described with reference to drawings. It should be understood that the embodiments described below are typical embodiments of the present invention and the scope of the present invention is not to be construed restricted by the embodiments.

### 1. Functions of cis element and cis element-binding factor

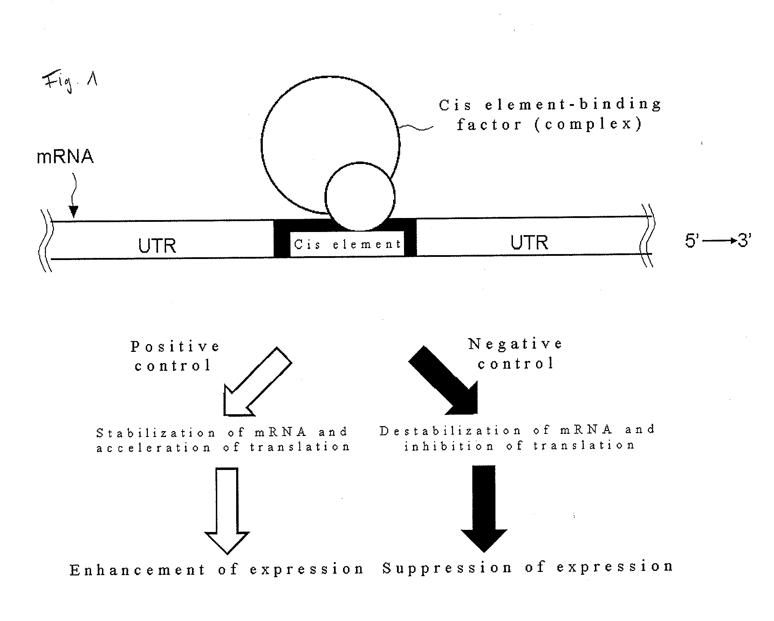

Figure 1 is a schematic chart explaining the mechanism of regulating the stability of mRNA and the amount of the mRNA translated into protein by a cis element and a cis element-binding factor.

A cis element-binding factor binds to a cis element present in 5' and 3' untranslated regions (5'UTR/3'UTR) in mRNA, forming a complex as a single factor or multiple factors, and regulates expression of the gene coded by the mRNA positively or negatively. In the case of positive control, the cis element-binding factor enhances gene expression by stabilizing the mRNA by inhibition of its decomposition and accelerating translation thereof into the protein. In contrast in the case of negative control, it suppresses gene expression by destabilizing the mRNA by acceleration of decomposition and thus inhibiting translation into the protein. For example, AUF1 (AU binding factor 1), an AU-rich element binding factor, binds to the ARE, forming a complex containing 3'-to-5' exoribonuclease, and accelerates decomposition of the mRNA (see Non patent Literature 3).

### 2. Polynucleotide and polynucleotide analogue

The polynucleotide or polynucleotide analogue according to the present invention, which has a nucleotide sequence complementary to a cis element in mRNA, binds to at least part of the cis element and thus inhibits the function, i.e., function to regulate expression positively or negatively, of the cis element-binding factor. Hereinafter, the configuration and the function of the polynucleotide and the polynucleotide analogue according to the present invention will be described specifically.

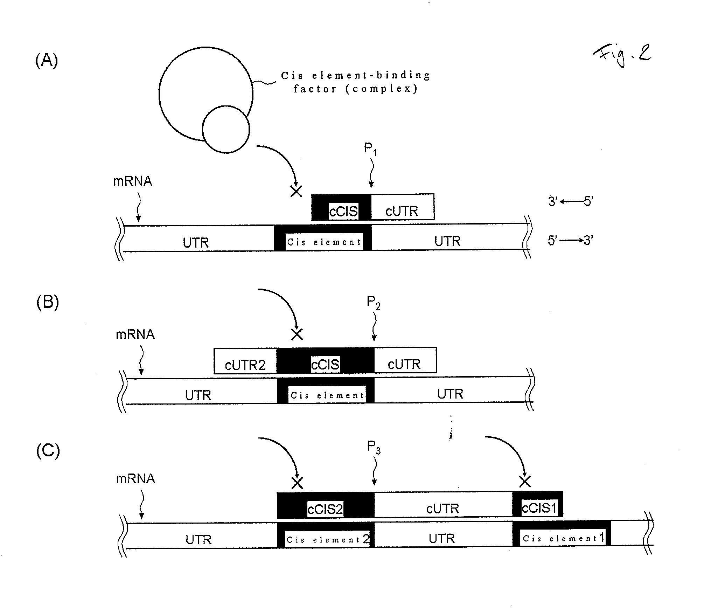

Figure 2 is schematic chart explaining an embodiment of the polynucleotide or polynucleotide analogue according to the present invention.

In Figure 2(A), the polynucleotide or polynucleotide analogue (hereinafter, referred to simply as "polynucleotide or the like") indicated by code P₁ has two continuing regions: a region "cCIS" having a nucleotide sequence complementary to part of the cis element present in the 5' or 3' untranslated region of mRNA and a region "cUTR" having a nucleotide sequence complementary to a 3'- untranslated region (3'-sided UTR) of the cis element.

When the polynucleotide or the like P₁ is a polynucleotide, cCIS and cUTR are DNA or RNA regions respectively having nucleotide sequences complimentary to those of the cis element and the 3'-sided UTR.

Alternatively when the polynucleotide or the like P₁ is a polynucleotide analogue, cCIS and cUTR are regions having one or both of the following regions: (1) nucleotide sequences complementary to the nucleotide sequences of cis element and 3'-sided UTR, or (2) nucleotide sequence-like structures of compounds that can recognize individually and bind complementarily to the bases, adenine, guanine, cytosine and uracil in the nucleotide sequences of cis element and 3'-sided UTR, which are aligned in nucleotide sequences mimicking the nucleotide sequences above.

The cUTR of the polynucleotide or the like P₁ has a function to bind the polynucleotide or the like P₁ to a particular cis element on a particular gene mRNA. Hereinafter, the particular gene mRNA and the particular cis element, to which the polynucleotide or the like P₁ binds, will be referred to respectively as "target gene mRNA" and "target cis element". Although a case where cUTR has a nucleotide sequence complementary to the 3'-sided UTR of a target cis element will be described below, the cUTR in the polynucleotide or the like P₁ may be designed to have a nucleotide sequence complementary to the 5'-sided UTR of the target cis element.

Each cis element such as AU-rich element (ARE) or Histone mRNA 3' UTR stem loop element has its characteristic nucleotide sequence. For example, a group of ARE (ARE I) has a nucleotide sequence characterized by the five bases of "AUUUA". Each cis element having such a particular nucleotide sequence is present on mRNAs of multiple genes and may be present in a plural number at different sites on the same mRNA. Thus, the cCIS of a polynucleotide or the like P₁ can bind to the target cis element of target gene mRNA and also to multiple cis elements on multiple gene mRNAs having a similar particular nucleotide sequence, based on complementarity.

On the other hand, the 3'-sided UTR of target cis element has a nucleotide sequence specific to the target cis element. The 3'-sided UTR of target cis element has a nucleotide sequence different from that of the 3'-sided UTRs of the cis element (other than the target) present at different sites on the same mRNA that have the particular nucleotide sequence of the target cis elements. In addition, the 3'-sided UTR of target cis element has a nucleotide sequence different from that of the 3'-sided UTRs of cis elements having the particular nucleotide sequence of the target cis elements present on the mRNAs of the genes other than the same target gene.

Thus, the cUTR of the polynucleotide or the like P₁ binds only to the 3'-sided UTR of target cis element specifically, based on the complementarity (sequence specificity) to the 3'-sided UTR of target cis element. It is possible to bind the cUTR-bound cCIS in the polynucleotide or the like P₁ only to the target cis element specifically by the bond specific to the cUTR.

The length (base number) of the cUTR of the polynucleotide or the like P₁ is a length giving a binding force sufficient for forming a double strand with the 3'-sided UTR of target cis element by the affinity due to hydrogen bonding between base pairs or the like. The length of the cUTR of the polynucleotide or the like P₁ is a length providing sufficiently high sequence specificity to the nucleotide sequence of the 3'-sided UTR of target cis element.

Traditionally in the fields of nucleic acid-related technologies such as PCR and DNA chip, designed was a polynucleotide that forms a double strand specifically only with a nucleotide strand containing a desired nucleotide sequence and is inhibited to bind to other nucleic acids non-specifically under a particular double strand-forming condition (hybridization condition). The length of the cUTR of the polynucleotide or the like P₁ in the present invention that is most favorable for sufficient binding force and sequence specificity can be determined, based on such a traditionally known polynucleotide-designing method.

Specifically, the length of the cUTR of the polynucleotide or the like P₁ is at least one base or more, preferably 2 bases or more, more preferably 3 bases or more and still more preferably 4 bases or more. However, the favorable length of the cUTR of the polynucleotide or the like P₁ varies, between when the polynucleotide or the like P₁ is a polynucleotide of DNA or RNA and when it is a polynucleotide analogue, for example containing LNA. For example, the length of cUTR may be shorter, when the polynucleotide or the like P₁ contains LNA higher in binding force and sequence specificity than when it contains DNA or RNA. The binding force and the sequence specificity of the cUTR of the polynucleotide or the like P₁ are influenced also by the entire length of the polynucleotide or the like P₁.

As described above, it is possible to bind cCIS in the polynucleotide or the like P₁ to the target cis element of target gene mRNA specifically. It is thus possible to inhibit binding of the cis element-binding factor to the target cis element competitively, by using the polynucleotide or the like P₁. It becomes possible to inhibit the function of the cis element-binding factor to regulate expression of mRNA and enhance or inhibit expression of the target gene, by inhibiting binding of the cis element-binding factor to the target cis element.

The length of the cCIS of the polynucleotide or the like P₁ is a length of the sequence that contains a sequence complementary to at least part of the target cis element and thus can inhibit binding of the cis element-binding factor by binding to the target cis element.

Specifically, the length of the CIS of the polynucleotide or the like P₁ is at least one base or more, preferably 2 bases or more, more preferably 3 bases or more and still more preferably 4 bases or more. It is considered that the cCIS can inhibit binding of the cis element-binding factor more effectively when it has larger length. However, increase in the length of cCIS may lead to increase in non-specific binding of the polynucleotide or the like P₁ and also to a problem in production cost.

The favorable length of the cCIS of the polynucleotide or the like P₁ is different between when the polynucleotide or the like P₁ is DNA or RNA and when it is a polynucleotide analogue for example of LNA, and the binding force of the cCIS of the polynucleotide or the like P₁ is influenced also by the entire length of the polynucleotide or the like P₁, as described above.

"Table 1" summarizes the functions of the target cis element and the cis element-binding factor binding thereto and the influence exerted when the binding is inhibited by the polynucleotide or the like P₁. (i) When the target cis element and the cis element-binding factor binding thereto regulates the target gene negatively, it is possible to stabilize the target gene mRNA selectively, accelerate translation of the target gene product and thus enhance expression of the target gene by inhibiting the binding with the polynucleotide or the like P₁. (ii) Alternatively when the target cis element and the cis element-binding factor binding thereto regulates the target gene positively, it is possible to destabilize the target gene mRNA selectively, inhibit translation of the target gene product and suppress expression of the target gene by inhibiting the binding with the polynucleotide or the like P₁.

**[Table 1]**

| Functions of target cis element and cis element-binding factor | Effect by inhibition of binding |
|---|---|
| (i) Negative control | Stabilization of mRNA/ acceleration |
| Destabilization of mRNA/inhibition | of translation |
| of translation | → Enhanced expression |
| (ii) Positive control | Destabilization of mRNA/inhibition |
| Stabilization of mRNA/acceleration | of translation |
| of translation | → Suppressed expression |

The effect of enhancement or suppression of gene expression can be obtained, even though any desired gene is used as the target gene, if the nucleotide sequences of the cCIS and the cUTR of the polynucleotide or the like P₁ are designed properly. Specifically, a particular cis element in a gene mRNA, of which expression is desirably regulated, is first determined and a cCIS having a nucleotide sequence complementary to that of the target cis element, i.e., cis element is designed. A cUTR complementary to the nucleotide sequence of the 3'-sided UTR of the target cis element is designed as a constituent of the polynucleotide or the like P₁. It is possible in this way to obtain the effect of enhancing and suppressing expression only of the target gene, as shown in "Table 1",by allowing the polynucleotide or the like P₁ bind only to the target cis element of the target gene mRNA, of which expression is desirably regulated.

The nucleotide sequences of the cCIS and the cUTR in the polynucleotide or the like P₁ need not be completely complementary to the nucleotide sequences of the target cis element and the 3'UTR thereof, and may contain bases (or compounds) non-complementary to one or more bases in the nucleotide sequences of the target cis element and the 3'UTR thereof, if they bind respectively to the target cis element and the 3'UTR thereof at high affinity, forming a double strand, and inhibit binding of the cis element-binding factor to the target cis element. In the present invention, such a nucleotide sequence will be called a "substantially equivalent nucleotide sequence".

Thus in the present invention, the "substantially equivalent nucleotide sequence" is a nucleotide sequence obtained by modification by deletion, addition, substitution or insertion of one or more (preferably, several or less) bases in the nucleotide sequence of the polynucleotide or the like, which binds to the target cis element and the 3'UTR thereof at high affinity, forming a double strand and has a function to inhibit binding of the cis element-binding factor to the target cis element, without any change in the function thereof.

The entire length of the polynucleotide or the like P₁ is determined by the sum of the lengths of the cUTR and the cCIS described above. The length of cUTR is a length providing sufficient binding force and sufficient sequence specificity to the 3'-sided UTR of target cis element, while the length of cCIS is a length of the sequence containing bases complementary to at least part of the target cis element and thus inhibiting binding of the cis element-binding factor by binding thereto by itself.

Specifically, the lengths of the cUTR and the cCIS in the polynucleotide or the like P₁ are respectively at least one base or more, preferably 2 bases or more, more preferably 3 bases or more and still more preferably 4 bases or more. Thus, the entire length of the polynucleotide or the like P₁ is at least two bases or more, preferably 4 bases or more, more preferably 6 bases or more and still more preferably 8 bases or more.

When the entire length of the polynucleotide or the like P₁ is longer, it has a higher binding force to the target cis element and the 3'UTR thereof. However, increase in entire length may lead to increase of non-specific binding of the polynucleotide or the like P₁ and also a problem in production cost.

In polynucleotide design in the field of nucleic acid-related technologies such as PCR and DNA chip, the desired length of the polynucleotide is several to dozens of bases, more preferably about 10 to 30 bases, for prevention of the problems of the non-specific binding and production cost. A most favorable value for the entire length of the polynucleotide or the like P₁ according to the present invention is determined, as such a traditionally known standard of polynucleotide design is taken into consideration. When the polynucleotide or the like P₁ contains LNA higher in binding force and sequence specificity, the entire length thereof may be shorter than the range above.

Hereinafter, other embodiments of the polynucleotide and the polynucleotide analogue according to the present invention will be described with reference to Figures 2(B) and 2(C).

The polynucleotide or the like according to the present invention may contain a region cCIS having a nucleotide sequence complementary to the entire length of the target cis element of target gene mRNA, similarly to the polynucleotide or the like indicated by code P₂ in Figure 2(B). As shown by the polynucleotide or the like P₂, it may have cUTR having a nucleotide sequence complementary to the 3'-sided UTR of the target cis element and also cUTR2 having a nucleotide sequence complementary to the 5'-sided UTR.

The length of the cUTR2 in the polynucleotide or the like P₂ is a length giving a binding force sufficient for forming a double strand with the 5'-sided UTR of the target cis element, by the affinity due to hydrogen bonding between base pairs or the like. In addition, the length of the cUTR2 in the polynucleotide or the like P₂ is a length providing sufficient sequence specificity to the nucleotide sequence of the 5'-sided UTR of the target cis element.

Specifically, similarly to the length of the cUTR of the polynucleotide or the like P₁ described above, it is at least one base or more, preferably 2 bases or more, more preferably 3 bases or more and still more preferably 4 bases or more. However in the case of the polynucleotide or the like P₂, the sequence specificity to the target cis element may be assured both with cUTR and cUTR2. Thus, the length of cUTR or cUTR2 in the polynucleotide or the like P₂ may be designed shorter than that of the cUTR of the polynucleotide or the like P₁.

The polynucleotide or the like according to the present invention may have regions cCIS1 and cCIS2 respectively having nucleotide sequences complementary to two cis elements 1 and 2 present in the target gene mRNA, as shown by the polynucleotide or the like indicated by code P₃ in Figure 2(C). In this case, cCIS1 and cCIS2 are connected to each other via cUTR. The cUTR has a nucleotide sequence complementary to the entire length of the UTR located at 5'-side of cis element 1 and 3'-side of cis element 2. The cis elements 1 and 2 may be one kind of cis element (for example, both AREs) or different kinds of cis elements (for example, one is ARE and the other IRES).

The length of the cCIS1 or the cCIS2 in the polynucleotide or the like P₃ is a length of the sequence containing bases complementary to at least part of the target cis element and allowing inhibition of binding of the cis element-binding factor, as it binds to the target cis element.

Specifically, the length of cCIS1 or cCIS2 in the polynucleotide or the like P₃ is at least one base or more, preferably 2 bases or more, more preferably 3 bases or more and still more preferably 4 bases or more, similarly to the length of the cCIS in the polynucleotide or the like P₁.

As described above, the polynucleotide or the like according to the present invention has a cCIS complementary to at least part of the target cis element and at least one cUTR complementary to the UTR located to the 5'- or 3'-side of the target cis element or the UTRs located to both sides (see Figures 2(A) to (C)). The polynucleotide or the like according to the present invention may have two or more cis elements in the target gene mRNA as targets (see Figure 2(C)).

### 3. Typical examples of polynucleotide analogues

As described above, when the polynucleotide or the like according to the present invention is a polynucleotide analogue, the cCIS or the cUTR may be a region having a nucleotide sequence-like structure, in which compounds capable of individually recognizing the bases, adenine, guanine, cytosine and uracil in the nucleotide sequences of cis element and 3'-sided UTR and binding thereto complementarily are aligned. In addition, in the polynucleotide analogue according to the present invention, the basic molecular chain of polynucleotide consisting of ribose molecules bound to each other with phosphodiester bonds may be replaced with a basic molecular chain in which ribose is modified, a basic molecular chain in which a sugar other than ribose is bound, or a basic molecular chain containing sugars other than ribose bound to each other by chemical bonds other than phosphodiester bonds.

For example, PNA, Morpholino, 2'-O-methyl-RNA, thio-DNA, thio-RNA,LNA or the like may be used, in preparation of the basic molecular chain in which ribose is modified, the basic molecular chain in which a sugar other than ribose is bound or the basic molecular chain containing sugars bound to each other by chemical bonds other than phosphodiester bonds.

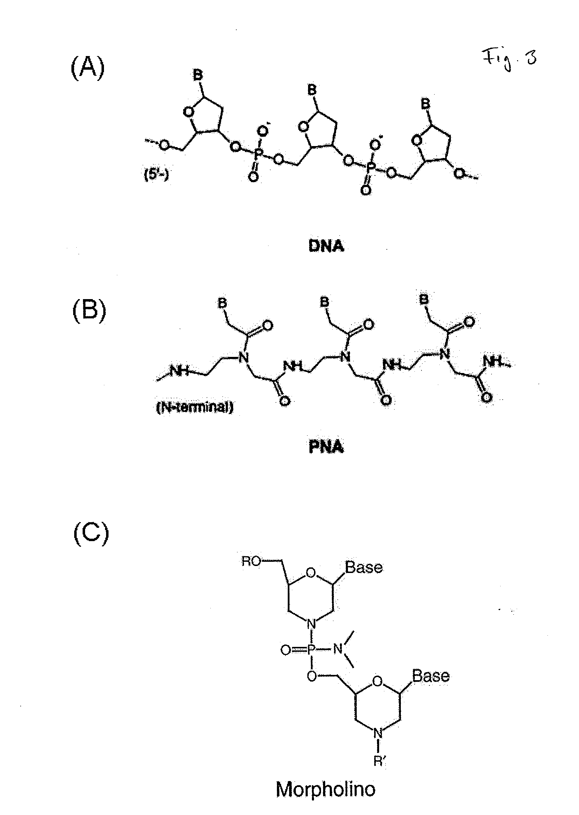

Figure 3 shows the binding structures respectively of PNA (B) and Morpholino (C). Figure 3(A) shows the binding structure of DNA. The PNA shown in Figure 3(B) is an example of the basic molecular chain of a polynucleotide analogue containing, in the basic molecular chain, non-sugars that are bound to each other via "-C(O)-NH-" bonds (amide bonds). Alternatively, the Morpholino shown in Figure 3(C) is an example of the basic molecular chain containing, in the basic molecular chain, non-sugars that are bound to each other via "-P(O)-O-" bonds (phosphomonoester bonds).

The polynucleotide analogue according to the present invention for use may be a combination of two or more of them above or a hybrid strand thereof with RNA or DNA. In particular, modified LNA in which the ribose structure is modified can be used favorably.

The oligonucleotide analogue prepared with LNA shows no "fluctuation" between two kinds of conformations, N- and S-type conformations, that are observed in oligonucleotides prepared with conventional natural nucleic acids and has an extremely high binding force to mRNA. Thus, for example, in the oligonucleotide analogue P₁ shown in Figure 2(A), it is possible to bind the cCIS to the target cis element more tightly, inhibiting binding of the cis element-binding factor to the target cis element effectively.

In addition, the oligonucleotide analogue prepared with LNA is also superior in sequence specificity. Thus, for example, in the oligonucleotide analogue P₁, it is possible to bind the cUTR to the 3'-sided UTR of the target cis element at higher specificity and increase the selectivity of the oligonucleotide analogue P₁ to the target element.

Because the oligonucleotide analogue prepared with LNA has high heat resistance and superior nuclease resistance, it is possible, if the oligonucleotide analogue is used as a gene product expression enhancer or inhibitor described below, to improve the stability and the effectiveness of the gene product expression enhancer or inhibitor.

The LNAs for use include 2',4'-LNAs in which 2'- and 4'-sites of the ribose are crosslinked with "-O-CH₂-" bonds and the conformation of which is fixed to N type, LNAs having a certain conformation fixed by various crosslink methods, and the like.

### 4. Typical examples of cis elements and cis element-binding factors

Examples of the target cis elements of the polynucleotide or the like according to the present invention include AU-rich elements, Histone mRNA 3' UTR stem loop element, Internal ribosome entry site (IRES), A2RE element, ZIPCODE element, Iron response element (IRE), Cytoplasmic polyadenylation (CPE), Nanos translational control, Amyloid precursor protein element (APP), Translational orientation element (TGE)/direct repeat element (DRE), Bruno element (BRE), 15-lipoxygenase differentiation control element (15-LOX-DICE), G-quartet element, Adh mRNA down-orientation element, Barley yellow dwarf virus, GLUT1 mRNA-stability control element, Msl-23 UTR control element, Msl-2 5 UTR control element, Ribosomal S12 mRNA translational control element, Selenocysteine insertion sequence type 1 (SECIS-1), Selenocysteine insertion sequence type 2 (SECIS-2), TNF-mRNA stability control element, Terminal oligopyrimidine tract (TOP), Vimentin mRNA 3 UTR control element and the like.

In particular, the target cis element is preferably an AU-rich element (ARE). The ARE and the ARE-binding protein regulate the stability and the translation amount of mRNA mostly negatively. It is thus possible, by inhibiting binding of the ARE-binding protein to ARE with the polynucleotide or the like according to the present invention and inhibiting the negative control function, to stabilize mRNA, accelerate translation thereof and amplify expression of the target gene (see "Table 1").

The cis element-binding factor, binding of which is inhibited by the polynucleotide or the like, may be for example a protein such as AUF1, HuR, Hel-N1, HuD, TTP, BRF1, TIA-1, KSRP, GUG-BP2, Nucleotin, TINO, PAIP2, ZFP36L1 or ZFP36L2, or a miRNA such as miR16.

The ARE is estimated to be present in 5 to 8% of all genes for proteins including cytokines such as IL-2, IL-3 and TNF-α, transcription factors and cell cycle-related proteins, and thus, it is possible to regulate expression of many genes having an important role in homeostasis, by using the ARE as the target cis element and by using the polynucleotide or the like according to the present invention.

### 5. Preparation of polynucleotide and polynucleotide analogue

The polynucleotide according to the present invention can be obtained by a known nucleic acid production method. For example when the polynucleotide analogue is prepared with LNA, it can be prepared by one of the known methods described in Patent Literatures 1 to 3.

### 6. Gene expression regulation method

In the gene expression regulation method using the polynucleotide or the like according to the present invention, when the system to be regulated is cell, it is possible, by adding the polynucleotide or the like to the cell culture solution and thus introducing it into the cells, to make it bound to the target cis element of target gene mRNA in the cells. Alternatively, the polynucleotide or the like may be introduced into cells by lipofection or microinjection, for binding thereof to the target cis element. When the system, of which gene expression is desirably regulated, is an organ or the body, the polynucleotide or the like is administered into the body or the organ therein and incorporated into the cells, through an administration route such as oral, enteral, nasal or blood vessel route or by direct local administration to the desired organ.

Yet alternatively, the polynucleotide may be bound to the target cis element of the target gene mRNA, by transfection of an expression vector and expression of the polynucleotide in the cells. The vectors for use include plasmids derived from Escherichia coli, Bacillus subtilis and yeasts, bacteriophages, retroviruses, vaccinia viruses, animal viruses such as baculovirus or the liposome fusion products thereof, and the like. In particular, when the polynucleotide is RNA, a retrovirus vector or the liposome fusion product thereof is selected favorably. The expression vector can be prepared by a known gene engineering method.

### 7. Target gene product expression enhancer or inhibitor and pharmaceutical composition

Hereinafter, the target gene product expression enhancer or inhibitor according to the present invention and the pharmaceutical composition containing the same as the active ingredient will be described.

The polynucleotide or the like according to the present invention can be used as an improver or inhibitor of target gene product expression. The gene product expression enhancer or inhibitor has a polynucleotide and/or polynucleotide analogue itself or an expression vector that can express the polynucleotide as the active ingredient.

The method of treating the target site, such as cell or organ, with the expression enhancer or inhibitor for use is selected most favorably from the methods described above: introduction of the polynucleotide or the like into cell by addition thereof to the cell culture solution or by lipofection, introduction thereof into an organ for example by oral administration, and expression thereof in cells by using an expression vector.

The pharmaceutical composition according to the present invention can be produced, by mixing the gene product expression enhancer or inhibitor with pharmaceutically allowable additives, such as carrier, flavor, diluent, vehicle, antiseptic, stabilizer and binder, in a unit dosage form needed for production of a generally accepted preparation. The pharmaceutical composition can be used, for example, orally as tablet as needed sugar-coated, capsule, elixir or microcapsule or parenterally as injection of an aseptic solution or suspension thereof in water or a pharmaceutically allowable liquid. Examples of the additives used as blended with the gene product expression enhancer or inhibitor according to the present invention for example in a raw tablet or capsule include binders such as gelatin, cone starch, tragacanth gum and Arabic gum; diluents such as crystalline cellulose; bulking agents such as cone starch, gelatin and alginic acid; lubricants such as magnesium stearate; sweeteners such as sucrose, lactose and saccharin; flavors such as peppermint, Akamono (Gaultheria adenothrx) oil and cherry; and the like.

The dosage, shape and administration method of the pharmaceutical composition are determined, as the age, body weight, symptom and others of the targeted administration object (including animal) are taken into consideration. The administration method is selected properly from indirect administration through oral, enteral, nasal or blood vessel route or direct local administration to a desired organ.

### 8. Regulation of LDLR expression

As will be described in detail in Examples below, the inventors have found that polynucleotide analogues (LNAs 1 to 3 and 6 to 14) respectively having the nucleotide sequences shown in "Table 2" can enhance distinctively expression of LDLR (low-density lipoprotein receptor) and that LNAs 1 to 3 and 6 to 14 enhance expression of the LDLR mRNA by binding to ARE1 (2790 to 2797 bases from the 5' terminal side in the nucleotide sequence of the LDLR mRNA represented by SEQ ID No. 4), inhibiting binding of ZFP36L1 and ZFP36L2, which functions to destabilize LDLR mRNA and accelerate decomposition thereof, to ARE, and selectively stabilizing and accelerating translation of the LDLR mRNA.

**[Table 2]**

| | | |
|---|---|---|
| LNA 1 | GAATAAATATA | (see SEQ ID No. 1) |
| LNA 2 | TCCCAGATGAATAAATATA | (see SEQ ID No. 2) |
| LNA 3 | AGATGAATAAA | (see SEQ ID No. 3) |
| LNA 6 | CTCCCAGATGA | (see SEQ ID No. 6) |
| LNA 7 | CCCAGATGAAT | (see SEQ ID No. 7) |
| LNA 8 | AAATATATAAA | (see SEQ ID No. 8) |
| LNA 9 | ATATATAAAAC | (see SEQ ID No. 9) |
| LNA 10 | AGATGAATA | (see SEQ ID No. 10) |
| LNA 11 | ATGAATAAA | (see SEQ ID No. 11) |
| LNA 12 | AGATGAAT | (see SEQ ID No. 12) |
| LNA 13 | ATGAATAA | (see SEQ ID No. 13) |
| LNA 14 | TGAATAAA | (see SEQ ID No. 14) |

Thus, a polynucleotide and polynucleotide analogue containing the nucleotide sequence represented by any one of SEQ ID Nos. 1 to 3 and 6 to 14 or a nucleotide sequence substantially equivalent to the nucleotide sequence, or alternatively an expression vector that can express the polynucleotide can be used as the LDLR expression enhancer.

The "polynucleotide having a substantially identical nucleotide sequence" is a polynucleotide having a modified sequence obtained by deletion, addition, substitution or insertion of one or more (preferably, several or less) bases of the nucleotide sequence represented by any one of SEQ ID Nos. 1 to 3 and 6 to 14, and showing a LDLR expression-enhanceing effect similar to that of the polynucleotide having the nucleotide sequence represented by any one of SEQ ID Nos. 1 to 3 and 6 to 14. Alternatively, the "polynucleotide analogue having a substantially identical nucleotide sequence" means a polynucleotide analogue having a sequence that can binds to the nucleotide sequences complementary thereto, similarly to the nucleotide sequence represented by any one of SEQ ID Nos. 1 to 3 and 6 to 14, i.e., a polynucleotide analogue, having a nucleotide sequence-like structure in which compounds individually recognizing adenine, guanine, cytosine and uracil are aligned and having a modified sequence obtained by deletion, addition, substitution or insertion of one or more (preferably, several or less) of the compounds, i.e., a polynucleotide analogue having a LDLR expression- enhanceing effect equivalent to that of the polynucleotide analogue having the nucleotide sequence represented by any one of SEQ ID Nos. 1 to 3 and 6 to 14.

The nucleotide sequence of the polynucleotide or the like is desirably completely equivalent to the nucleotide sequence represented by any one of SEQ ID Nos. 1 to 3 and 6 to 14 for high LDLR expression-enhanceing action, but may not be identical completely therewith, and may be a nucleotide sequence substantially equivalent to the nucleotide sequence represented by any one of SEQ ID Nos. 1 to 3 and 6 to 14, if it is a nucleotide sequence binding to the target ARE of LDLR mRNA at high affinity and forming a double strand. The polynucleotide or the like having such a modified sequence may be designed, for example, for prevention of adverse reactions generated by binding of other genes to mRNAs or for improvement of the cell membrane-permeability and the in-vivo stability of the polynucleotide or the like.

The LDLR has a function to incorporate LDL cholesterol in blood plasma into cell by endocytosis via the receptor. In particular, incorporation of LDL cholesterol into cell by the LDLR expressed in liver plays an important role in clearance of plasma LDL cholesterol.

LDL cholesterol is involved in transportation of cholesterol from liver to peripheral tissues. However in the state of hyperlipidemia, where the blood LDL cholesterol is higher than its normal value, the LDL cholesterol leads to damage of the blood vessel by deposition thereof on an inner wall of the blood vessel, possibly causing diseases such as arteriosclerosis, hypertension, cerebral infarction, myocardial infarction and angina cordis.

The LDLR expression enhancer according to the present invention can enhance expression of LDLR selectively, and it is possible, with a pharmaceutical composition containing the LDLR expression enhancer as the active ingredient, to accelerate clearance of plasma LDL cholesterol and prevent, alleviate or treat hyperlipidemia and hyperlipidemia-related diseases in particular by accelerating expression of LDLR in liver.

The genetic polymorphism of LDLR is reported to be related with the risk of developing Alzheimer's disease (see "Function of beta-amyloid in cholesterol transport: a lead to neurotoxicity." FASEB J., 2002, Oct; 16 (12): 1677-9. Epub 2002 Aug 21.). It is also suggested that increase of cholesterol level has something to do with the condition of Alzheimer's disease (see "Genetic study evaluating LDLR polymorphisms and Alzheimer's disease." Neurobiol Aging., 2008, Jun; 29 (6): 848-55. Epub 2007 Jan 18.).

The results show that the LDLR expression enhancer and the pharmaceutical composition according to the present invention enhance expression of LDLR selectively and regulate the level of plasma LDL cholesterol, suggesting a possibly of it being applied to prevention, alleviation, or treatment of Alzheimer's disease.

The term "prevention" includes not only prevention before development of disease, but also prevention of recurrence after treatment of the disease. The hyperlipidemia-related diseases include, in addition to arteriosclerosis, hypertension, cerebral infarction, myocardial infarction and angina cordis described above, diabetes, adiposis and cancer, but application to other diseases is not excluded.

### Examples

### <Example 1>

### 1. Regulation of LDLR expression I (ARE1)

The polynucleotide and the polynucleotide analogue according to the present invention having LDLR as the target gene and the gene expression regulation method by using the same were evaluated.

### (1) Design of polynucleotide analogue

A polynucleotide analogue complementary to part of ARE on LDLR mRNA was designed with reference to an ARE database (AU-RICH ELEMENT-CONTAINING mRNA DATABASE: http://brp.kfshrc.edu.sa/ ARED/).

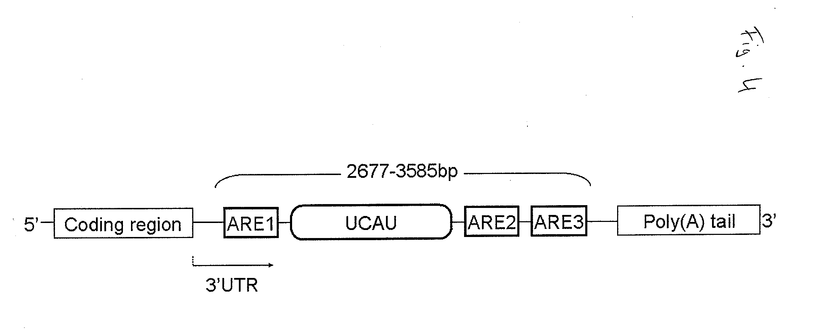

It was known hitherto that at least three AREs were present in the 3'UTR of LDLR mRNA (see "Berberine is a novel cholesterol-lowering drug working through a unique mechanism distinct from statins." Nat Med., 2004, Dec; 10 (12): 1344-51. Epub 2004 Nov 7.). "Figure 4" is a schematic chart showing the locations of these three AREs in the 3'UTR on LDLR mRNA. The three AREs will be formally referred to as "ARE1", "ARE2" and "ARE3" (the "AREs 1 to 3" are different from the three groups of AREs, "AREs I to III" described above). In the Figure, "UCAU" represents "UCAU repeat" having a sequence of four repeated bases of "UCAU".

AREs 1 to 3 are located in the region of 2677-3585 bp from the 5' terminal of the LDLR mRNA 5175 bp (GenBank Accession No. NM_000527) represented by SEQ ID No. 4. ARE1 corresponds to the region of 2790 to 2797 bps from the 5' terminal, ARE2 to the region of 3350 to 3363 bps from the 5' terminal, and ARE3 to the region of 3538 to 3547 bps from the 5' terminal.

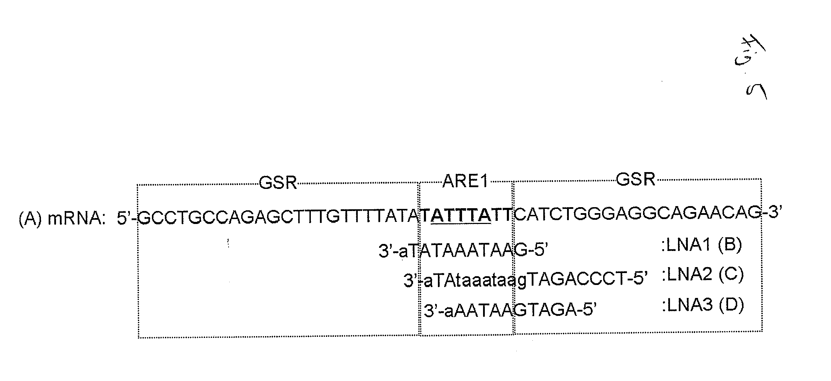

Figure 5 shows the nucleotide sequence of a polynucleotide analogue designed by using ARE1 as the target cis element and the partial nucleotide sequence of the ARE1-containing LDLR mRNA region.

Figure 5(A) shows the nucleotide sequence of an ARE1-containing region (2767-2816 bps from 5'- terminal) in the LDLR mRNA having an entire length of 5175 bps (see SEQ ID No. 4). The region indicated by bold letters in the nucleotide sequence represents ARE1, while the region underlined represents "AUUUA pentamer".

Figures 5(B) to 5(D) shows polynucleotide analogues having a nucleotide sequence complementary to part of ARE 1. The region in the nucleotide sequence indicated by capital letters represents LNA and that indicated by lower case letters represents RNA. Each of the polynucleotide analogues represented by Figures 5(B) to 5(D) is a hybrid nucleic acid that is prepared from LNA and RNA. Hereinafter, they are respectively referred to as LNA1 to LNA3. LNA1 to LNA3 were obtained by contracted preparation (Gene Design).

LNA1 shown in Figure 5(B) has a nucleotide sequence containing "AUUUA pentamer" that is complementary to the entire ARE and nucleotide sequences complementary to part of 5'UTR and 3'UTR connected to the ARE1 on LDLR mRNA (see SEQ ID No. 1). The length of LNA1 is 11 bps, and 5'-sided first to 10th bases are prepared from LNA and the 11th base from RNA. The first nucleotide sequence from the 5' side and the first to second nucleotide sequence from the 3' side correspond to the ARE1-specific nucleotide sequence (Gene specific region: GSR) of LDLR gene. See the UTR described in Figure 2 for GSR.

LNA2 shown in Figure 5(C) has a nucleotide sequence complementary to the entire ARE1 containing the "AUUUA pentamer" and a nucleotide sequence complementary to part of the 3'UTR connected to ARE of LDLR mRNA (see SEQ ID No. 2). The length of LNA2 is 19 bps, and the first to 8th and 17th to 18th bases from the 5' side are prepared from LNA and the 9th to 16th and the 19th bases from RNA. The first to 9th nucleotide sequence form the 5' side corresponds to GSR of ARE1 in the LDLR gene.

LNA3 shown in Figure 5(D) has a nucleotide sequence complementary to part of ARE1 and a nucleotide sequence complementary to part of the 3'UTR connected to ARE1 of LDLR mRNA (see SEQ ID No. 3). The length of LNA3 is 11 bps, and the first to 10th bases from the 5' side is prepared from LNA and the 11 th base from RNA. The first to 5th nucleotide sequence from the 5' side corresponds to GSR of ARE1 in the LDLR gene.

### (2) Transfection of polynucleotide analogue

Hela cells were inoculated on a 6-well plate at a concentration of 4.0×105 cells/well and cultured in 10% FBS-containing DMEM medium. After culture for 24 hours, a polynucleotide analogue was transfected into the cells by lipofection (Lipofectamine 2000, Cat. No. 11668-019, Invitrogen Corporation). 200 pmol of the polynucleotide analogue was diluted respectively with 100 µl of Opti-MEM. Separately, 10 µl of Lipofectamine 2000 was diluted with 100 µl of Opti-MEM. After dilution, the mixture was left still at room temperature for 5 minutes; the diluted polynucleotide analogue solution was mixed with the diluted lipofectamine solution; the mixture was left still additionally for 20 minutes; and the entire amount was transferred into each well on the 6-well plate.

In addition to the LNAs 1 to 3 above, the following LNA4 and LNA5 shown in "Table 3" were used as controls for the polynucleotide analogues. These compounds are polynucleotide analogues having a nucleotide sequence not complimentary to the LDLR mRNA at all.

**[Table 3]**

| Polynucleotide analogue | Nucleotide sequence |
|---|---|
| LNA4 | TGAGAGCTTGa |
| LNA5 | AGCTGTAACAc |

In the Table above, the region indicated by capital letters represents LNA, while the region indicated by lower case letters RNA.

### (3) Evaluation of LDLR expression level

The cells 24 hours after transfection were collected and the protein therein was extracted by a common method. 5 µg of the extracted protein was separated by SDS-PAGE and blotted on a membrane. The expression level of LDLR was evaluated by Western blotting according to a common method. An antibody of Abcam plc. (Cat. No. ab52818) was used as the anti-LDLR antibody.

### (4) Results

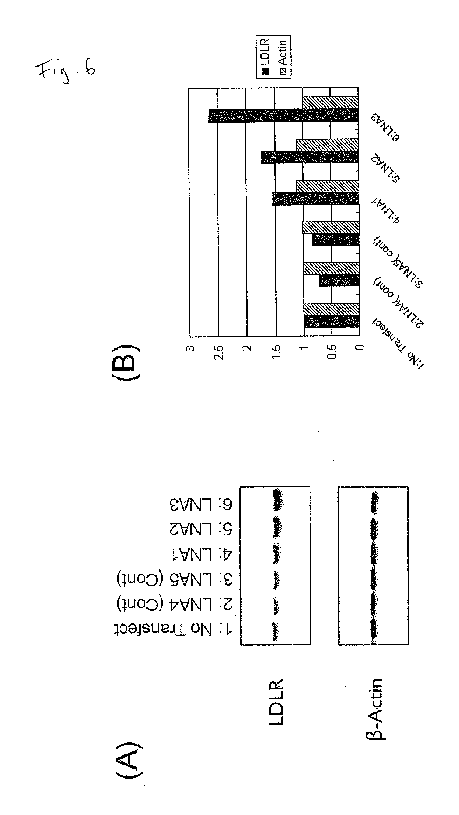

The results of Western blotting are shown in "Figure 6". The top photo in Figure (A) shows the band of LDLR detected and the bottom photo thereof the band of β-actin for comparison detected. An antibody of Cell Signaling Technology, Inc. (Cat. No. 4970) was used as the anti-β-actin antibody. Figure (B) shows the quantitative values of the concentrations of the detected bands, as relative rates compared to 1 of the concentrations in the cells without transfection (lane 1 in Figure (A)).

Compared to the cells without transfection of the polynucleotide analogue (lane 1 in Figure (A)) and the cells for control that were transfected respectively with LNA4 and LNA5 (lanes 2 and 3), the cells transfected respectively with LNAs 1 to 3 (lanes 4 to 6) were found to show increase in LDLR expression level.

The effectiveness of enhanceing LDL expression level was larger in the order of LNA3 > LNA2 > LNA1. It is probably because of the following reasons:

Because LNA1 has a shorter nucleotide sequence corresponding to the GSR in the LDLR gene, it can bind to ARE in other gene mRNA having the nucleotide sequence equivalent to ARE 1. Thus, the amount of the polynucleotide analogue selectively bound to the LDLR mRNA is smaller in the case of LNA1, probably because the effectiveness of enhancing LDLR expression was smaller, compared with that by LNA2 or LNA3.

In contrast, LNA2, wherein the first to 9th nucleotide sequence from the 5' side is a nucleotide sequence corresponding to GSR of ARE 1 in the LDLR gene, can bind to ARE1 in the LDLR mRNA specifically, based on its sequence specificity. It is thus considered that LDLR-selective enhancement of gene expression proceeded with LNA2 and an effectiveness higher than that by LNA1 was obtained.

Alternatively in the case of LNA2, a nucleotide sequence complementary to the 10th to 16th ARE1 from the 5' side is prepared from RNA, while in the case of LNA3, the entire length is prepared from LNA (however, excluding 3' terminal). For that reason, LNA3 shows a nuclease resistance higher than LNA2. In addition, the nucleotide sequence complementary to ARE1 of LNA3 prepared with LNA is higher in binding force to ARE1 and also in sequence specificity than the same sequence of LNA2 prepared with RNA. It was therefore considered that LNA3 binds to ARE1 more efficiency and tightly than LNA2, showing higher LDLR expression- enhanceing effect.

On the other hand, in the case of β-actin, there was no significant change in expression level, independently of the presence or absence of transfection or the kind of the transfected LNA in all cells.

The results above showed that it is possible to increase the expression level of the target gene LDLR specifically with LNAs 1 to 3.

### <Example 2>

### 2. Identification of ARE-binding factor

The results in Example 1 suggested that LNAs 1 to 3 enhances LDLR expression level selectively by binding to ARE1 in the LDLR gene specifically, inhibiting binding of the ARE-binding factor to ARE 1, and thus inhibiting the negative expression-regulating function of the ARE-binding factor. Thus, effort was made then to identify the ARE-binding factor, of which binding to ARE1 is inhibited by LNAs 1 to 3 and to analyze the functions thereof. First for identification of the ARE-binding factor binding to ARE1, cis element-binding proteins binding to the cis element in LDLR mRNA were analyzed comprehensively by immunoprecipitation using LDLR mRNA as a bait and proteome analysis using mass spectrometer.

### (1) Preparation of LDLR mRNA bait

LDLR mRNA was prepared by in-vitro translation. A region of 2677 to 3585 bps in LDLR mRNA (SEQ ID No. 4, GenBank Accession No. NM_000527) was enhanced by PCR by using a primer having a T7 promoter sequence at the 5' terminal, and the RNA was prepared by using MEGAscript T7 kit (Cat. No. 1333, Ambion Inc) according to the attached protocol. The 3' terminal of the LDLR mRNA was labeled with a flag, in covalent bond-forming reaction of the 3' terminal of the LDLR mRNA prepared with a flag hydrazide. The labeled mRNA was purified by using a RNeasy Mini Kit (Cat. No. 74106) of QIAGEN. The flag labeling of the mRNA was carried out by a known method (see "Programmable ribozymes for mischarging tRNA with nonnatural amino acids and their applications to translation." Methods, 2005, Vol. 36, No. 3, p. 239-244).

### (2) Immunoprecipitation and identification of proteins

10 pmol of the flag-labeled LDLR mRNA after purification was mixed with anti-flag antibody beads (Cat. No. F2426, SIGMA CORPORATION) and the mixture was allowed to react at 4°C for 1 hour. 3 mg of a cell extract protein, which was extracted from 293T cells cultured in 10% FBS-containing DMEM medium was then added thereto, and the mixture was allowed to react at 4°C additionally for 1 hour. After wash out of the non-binding proteins, RNAs and RNA-binding proteins were eluted with the flag peptide. The sample obtained by elution was treated with lysyl endopeptidase, and the product was analyzed by using a known method, LC-MS/MS method (see "A direct nanoflow liquid chromatography-tandem mass spectrometry system for interaction proteomics." Analytical Chemistry, 2002, Vol. 74, No. 18, p. 4725-4733). The mass spectrometer used was QSTAR XL (Applied Biosystems).

### (3) Results

"ZFP36L1" and "ZFP36L2" were identified by LC-MS/MS. ZFP36L1 and ZFP36L2 constitute a family of ARE-binding factors (hereinafter, referred to as "ZFP36 family"), together with ZFP36 (alias TTP). The ZFP36 family is reported to have functions to bind to ARE, destabilize the mRNA and accelerate decomposition thereof (see "Tristetraprolin and its family members can promote the cell-free deadenylation of AU-rich element-containing mRNAs by poly (A) ribonuclease." Molecular Cell Biology, 2003, Vol. 23, No. 11, p. 3798-812). Thus, the results strongly suggested that the ARE-binding factors having the negative expression-regulating function described in Example 1, binding of which to ARE1 can be inhibited by LNAs 1 to 3 were ZFP36L1 and ZFP36L2.

### <Example 3>

### 3. Evaluation of binding potential of ZFP36L1 and ZFP36L2 to LDLR mRNA and binding-inhibiting potential of LNAs 1 to 3

Studied were performed whether ZFP36L1 and ZFP36L2 can bind to 3'UTR of LDLR mRNA and whether LNAs 1 to 3 can inhibit binding of the ZFP family to 3'UTR.

3'UTR of LDLR mRNA containing ARE1 was prepared by in-vitro translation and labeled with a flag peptide, by the method described in "(1) Preparation of LDLR mRNA bait" in Example 2. The polynucleotide of 2677-3585 bps from the 5'- terminal in the LDLR mRNA (see SEQ ID No. 4) was used as 3'UTR. For comparison, 3'UTR of beta-Actin mRNA (1199-1770 bps from 5'- terminal) (SEQ ID No. 5, GenBank Accession No. NM_001101) was also prepared similarly.

The flag-labeled LDLR 3'UTR after purification (hereinafter, referred to as "LDLR 3'UTR-flag") or the flag-labeled Actin 3'UTR (hereinafter, referred to as "Actin 3'UTR-flag") was mixed with a cell extract protein extracted from the 293T cells that were forced to express ZFP36L1 and ZFP36L2 fused with Myc protein (Myc-tagged-ZFP36L1/ZFP36L2); additionally, one of LNAs 1 to 5 (see Figure 3 and Table 2) was added thereto, to a final concentration of 0, 30, or 100µm; and the mixture was allowed to react, by the method described in "(2) Immunoprecipitation" of Example 2. After immunoprecipitation, the eluted sample was subjected to Western blotting by using an anti-Myc antibody (Cat. No. 1667149, Roche Diagnostics K.K.).

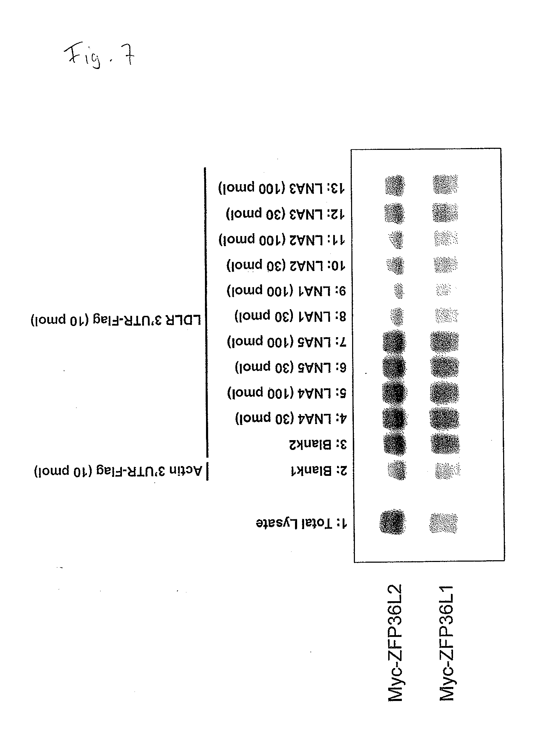

The results obtained by the Western blotting are shown in "Figure 7". The top band represents a Myc-tagged-ZFP36L2 band detected with an anti-Myc antibody, while the bottom band represents a Myc-tagged-ZFP36L1 band.

A sample obtained by mixing a cell extract protein containing Myc-tagged-ZFP36L1/ZFP36L2 with LDLR 3'UTR-flag and immunoprecipitating the product with an anti-flag antibody gave ZFP36L1 and a great amount of ZFP36L2 detected (lane 3 in the Figure). On the other hand, a sample obtained in reaction with Actin 3'UTR-flag gave extremely small detection signals of ZFP36L1 and ZFP36L2 (lane 2).

The results indicate that Myc-tagged-ZFP36L1/ZFP36L2 can bind to LDLR 3'UTR-flag and ZFP36L1 and ZFP36L2 have functions to bind LDLR to 3'UTR.

Detection signals of ZFP36L1 and ZFP36L2 were distinctively lower in lanes 8 to 13, where the cell extract protein and the LDLR 3'UTR-flag were allowed to react in the presence of one of LNAs 1 to 3 having a nucleotide sequence complementary to ARE1 of LDLR mRNA. The intensity of the detection bands in lanes 8 to 13 was not larger than that of the detection bands obtained in the reaction with an Actin 3'UTR-flag, as shown in lane 2. On the other hand, there was no decrease observed in the detection signals of ZFP36L1 and ZFP36L2 in lanes 4 to 7, where the reactions were carried out in the presence of control LNAs 4 and 5.

The results show that ZFP36L1 and ZFP36L2 bind particularly to ARE1 in LDLR mRNA 3'UTR and LNAs 1 to 3 can inhibit binding of ZFP36L1 and ZFP36L2 to the target ARE distinctively.

### <Example 4>

### 4. Functional analysis of ZFP36L1 and ZFP36L2

Change in the expression level of LDLR when expression of ZFP36L1 and ZFP36L2 was inhibited by using RNAi was analyzed, to elucidate the functions of ZFP36L1 and ZFP36L2 in regulation of LDLR expression.

Hela cells were inoculated on a 6-well plate at a concentration of 4.0×10⁵ cells/well and cultured in 10% FBS-containing DMEM medium. After culture for 24 hours, siRNA was transfected into the cells by lipofection (Lipofectamine 2000, Cat. No. 11668-019, Invitrogen Corporation). Polynucleotide analogues in amounts respectively of 200 pmol were diluted with 100 µl of Opti-MEM. Separately, 10 µl of Lipofectamine 2000 was diluted with 100 µl of Opti-MEM. The solution was left still at room temperature for 5 minutes after dilution, the diluted polynucleotide analogue solution and the diluted Lipofectamine solution were mixed with each other, and the resulting solution was left still additionally for 20 minutes and then, the entire amount thereof was poured into the wells of the 6-well plate.

The siRNA used was purchased from Invitrogen Corporation. Cat. Nos. of the three kinds of siRNAs used for analysis of ZFP36L1 and ZFP36L2 are shown in "Table 4". The control siRNA7-9 used was Stealth RNAi Negative Control (Cat. No. 12935-100, Invitrogen Corporation).

**[Table 4]**

| siRNA | Target | Cat. No. |
|---|---|---|
| siRNA 1 | ZFP36L1 | HSS101101 |
| siRNA 2 | ZFP36L1 | HSS101102 |
| siRNA 3 | ZFP36L1 | HSS101103 |
| siRNA 4 | ZFP36L2 | HSS101104 |
| siRNA 5 | ZFP36L2 | HSS101105 |
| siRNA 6 | ZFP36L2 | HSS101106 |

The cells after 60 hours from transfection were collected, and the protein extracted from the cells was analyzed by Western blotting, for evaluation of the expression level of LDLR.

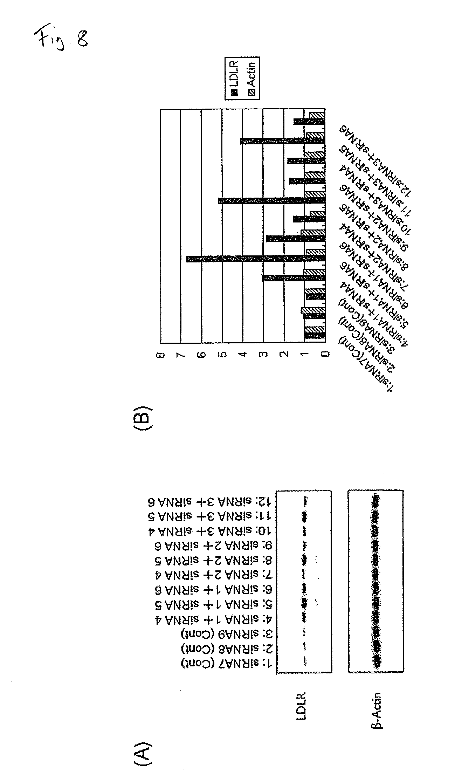

The results obtained by Western blotting are shown in "Figure 8". The top photo of Figure (A) shows the detection band of LDLR, while the bottom photo shows the detection band of β-actin for comparison. Figure (B) shows quantitative values of the concentrations of detected bands, as relative rates compared to 1 of the concentrations in the cells transfected with control siRNA7 (lane 1 in Figure (A)).

Compared to the cells transfected with control siRNAs 7 to 9, cells transfected with a combination of any one of siRNAs 1 to 3 and any one of siRNAs 4 to 6, in which expression of ZFP36L1 and ZFP36L2 was inhibited, had a distinctively increased expression level of LDLR.

On the other hand, as for β-actin, there was no significant change in its expression level, even when the cells were transfected with siRNAs 1 to 6.

The fact that transfection of siRNA, which inhibits expression of ZFP36L1 and ZFP36L2, leads to distinctively increase in expression level of LDLR, as described above, indicates that ZFP36L1 and ZFP36L2 function to destabilize LDLR mRNA and accelerate decomposition thereof, thus regulating LDLR expression negatively.

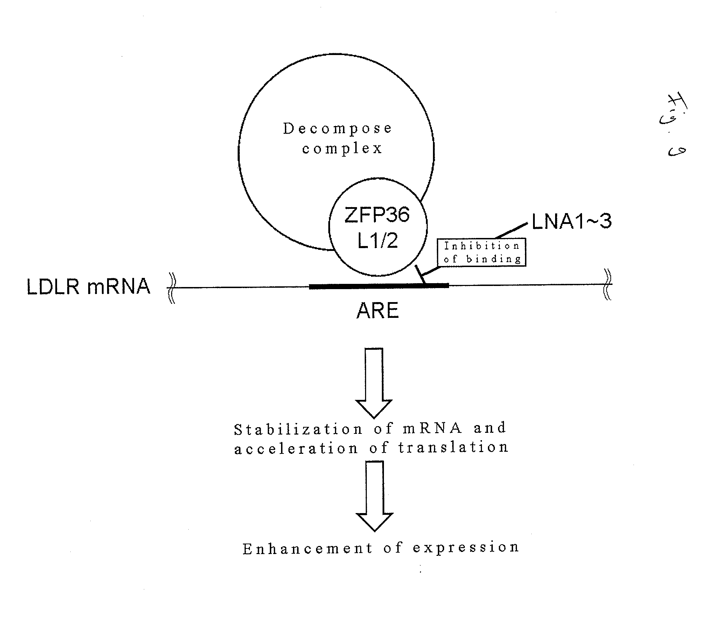

The results in Examples 1 to 4 showed that LNAs 1 to 3 inhibit binding of ZFP36L1 and ZFP36L2 to ARE1 by binding to ARE1 of LDLR mRNA and that the binding thereof to ARE1 leads to inhibition of the negative gene expression-regulating function of ZFP36L1 and ZFP36L2, stabilization of LDLR mRNA and acceleration of translation, and thus to selective acceleration of expression of LDLR mRNA (see Figure 9).

### <Example 5>

### 5. Regulation of LDLR expression II (AREs 2 and 3)

Subsequently, a LDLR expression regulation experiment similar to that in Example 1 was carried out, by using ARE2 and ARE3, among the three AREs present in 3'UTR of LDLR mRNA (see Figure 4), as the target cis elements.

### (1) Design of polynucleotide analogue

The nucleotide sequences of the polynucleotide analogues designed by using ARE2 or ARE3 as the target cis element are summarized in "Table 5". LNA26 has a nucleotide sequence complementary to ARE2 (3350 to 3363 bps from the 5'- terminal) of LDLR mRNA having an entire length of 5175 bps (see SEQ ID No. 4). Alternatively, LNA27 has a nucleotide sequence complementary to ARE3 (3538-3547 bps from the 5'- terminal).

**[Table 5]**

| Polynucleotide analogue | Nucleotide sequence | Length (bps) |
|---|---|---|
| LNA26 | CACTTAATAAA | 11 |
| LNA27 | ATAATAACACA | 11 |

LNA26 or LNA27 was transfected into Hela cells by lipofection in a manner similar to Example 1. The cells 24 hours after transfection were collected, and the protein was extracted and subjected to Western blotting, for evaluation of the expression level of LDLR.

### (2) Results

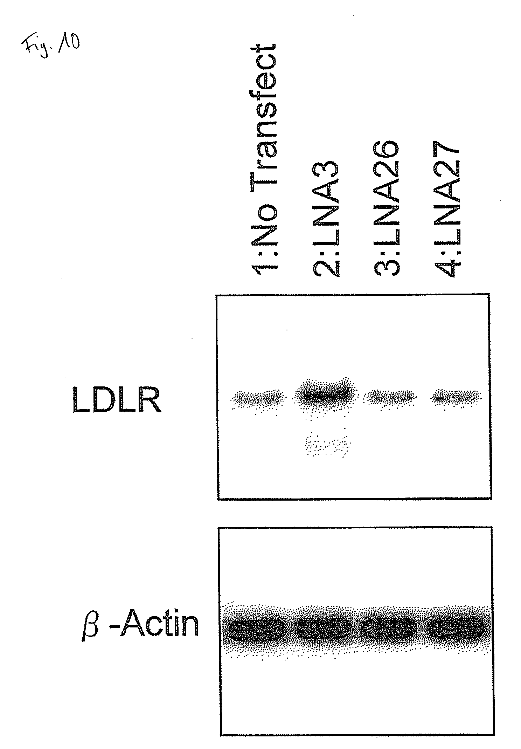

Results obtained by the Western blotting are shown in "Figure 10". In the Figure, the top photo shows the band of LDLR detected, while the bottom photo shows the band of β-actin for comparison detected.

The LNA3-transfected cells used in Example 1 (lane 2) were found to be significantly increased in the expression level of LDLR, compared to the cells without transfection of the polynucleotide analogue (lane 1). In contrast, the cells transfected with LNA26 and LNA27 (lanes 3 and 4) were not found to be increased in expression level of LDLR. As for β-actin, there was no significant change in expression level, independently of the presence or absence of transfection or the kind of the transfected LNA in all cells.

### <Example 6>

### 6. Evaluation of binding potential of ZFP36L1 and ZFP36L2 to ARE2 or ARE3

It was examined in the present Example whether the ZFP family can bind to ARE2 or ARE3.

### (1) Preparation of LDLR mRNA bait

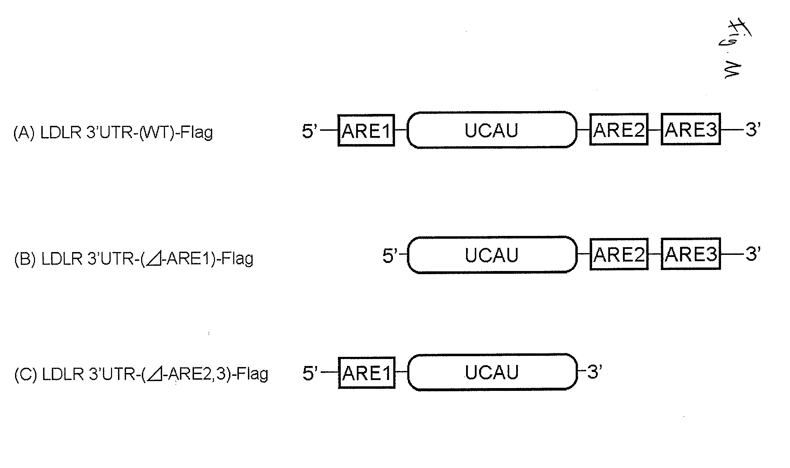

Baits respectively lacking AREs 1 to 3 were prepared and the binding potentials thereof of binding ZFP36L1 and ZFP36L2 to AREs 1 to 3 were evaluated. The structures of the mRNA baits prepared were shown in "Figure 11". Figure 11(A) shows a mRNA bait (LDLR 3'UTR-(WT)-flag) containing the 2677-3585 bp region of LDLR mRNA. Alternatively, Figures (B) and (C) show respectively a mRNA bait lacking ARE1 region (LDLR 3'UTR-(Δ-ARE1)-flag) and a mRNA bait lacking ARE2 and ARE3 (LDLR 3'UTR-(Δ-ARE2,3)-flag). The mRNA baits were prepared according to the method described in "(1) Preparation of LDLR mRNA bait" of Example 2.

### (2) Immunoprecipitation

According to the method described in Example 3, the flag-labeled mRNA after purification was mixed with anti-flag antibody beads for reaction and 3 mg of cell extract protein extracted from 293T cells that were forced to express Myc-tagged-ZFP36L1/ZFP36L2 were mixed therewith for reaction. The sample obtained by elution after immunoprecipitation was analyzed by Western blotting, by using an anti-Myc antibody (Cat. No. 1667149, Roche Diagnostics K.K.).

### (3) Results

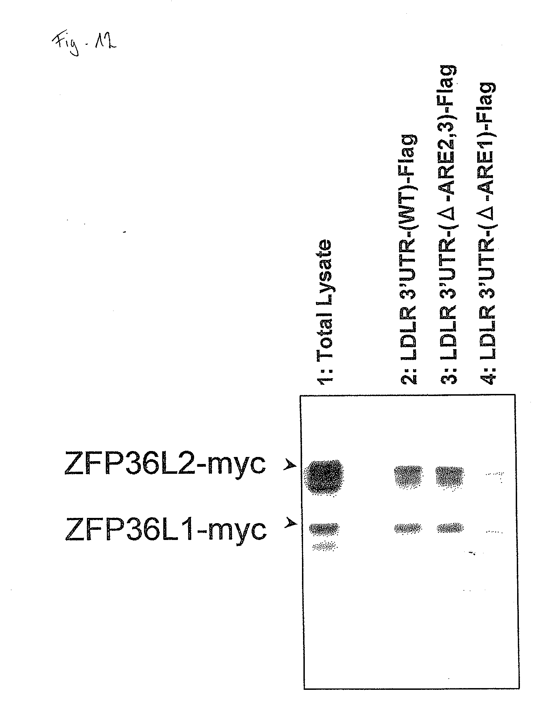

Results obtained by Western blotting are shown in "Figure 12". The top band represents a Myc-tagged-ZFP36L2 band detected with the anti-Myc antibody, while the bottom band represents a Myc-tagged-ZFP36L1 band.

The sample obtained by reaction with LDLR 3'UTR-(Δ-ARE1)-flag lacking ARE1 (lane 4) gave almost no ZFP36L1 and ZFP36L2 detected. The results indicate that ZFP36L1 and ZFP36L2 bind particularly to ARE1 and not bind to ARE2 and ARE3.

In addition, the sample obtained by mixing the LDLR 3'UTR-(Δ-ARE2,3)-flag lacking ARE2 and ARE3 with the cell extract protein containing Myc-tagged-ZFP36L1/ZFP36L2 and immunoprecipitating the product with an anti-flag antibody (lane 3 in the Figure) gave ZFP36L1 and ZFP36L2 detected in amounts almost equivalent to those observed with the LDLR 3'UTR-(WT)-flag containing the entire region. The results support the finding that ZFP36L1 and ZFP36L2 bind to ARE1.

### <Example 7>

### 7. Regulation of LDLR expression III (ARE1)

In the present Example, several kinds of polynucleotide analogues were designed by using ARE1 as the target cis element for enhancement of LDLR expression (see Figure 9).

"Table 6" shows the nucleotide sequences of the polynucleotide analogues (LNAs 6 to 25) newly designed. In the nucleotide sequences above, the underlined region indicates a nucleotide sequence complementary to ARE1 (see also Figure 5).

**[Table 6]**

| Polynucleotide analogue | SEQID No. | Nucleotide sequence | | | Length (bps) |
|---|---|---|---|---|---|
| LNA3 | 3 | AGATG | AATAAA | | 11 |
| LNA6 | 6 | CTCCCAGATG | A | | 11 |
| LNA7 | 7 | CCCAGATG | AAT | | 11 |
| LNA8 | 8 | | AAATA | TATAAA | 11 |
| LNA9 | 9 | | ATA | TATAAAAC | 11 |
| LNA10 | 10 | AGATG | AATA | | 9 |
| LNA11 | 11 | ATG | AATAAA | | 9 |
| LNA12 | 12 | AGATG | AAT | | 8 |
| LNA13 | 13 | ATG | AATAA | | 8 |
| LNA14 | 14 | TG | AATAAA | | 8 |
| LNA15 | - | | AATAAATA | | 8 |
| LNA16 | - | | AAATA | TAT | 8 |
| LNA17 | - | | | TATAAAAC | 8 |
| LNA18 | - | AGATG | AA | | 7 |
| LNA19 | - | GATG | AAT | | 7 |
| LNA20 | - | ATG | AATA | | 7 |
| LNA21 | - | | AATAAAT | | 7 |
| LNA22 | - | | AAATA | TA | 7 |
| LNA23 | - | | | TATAAAA | 7 |
| LNA24 | - | CTGCCTCCCAG | | | 11 |
| LNA25 | - | GCCTCCCAGAT | | | 11 |

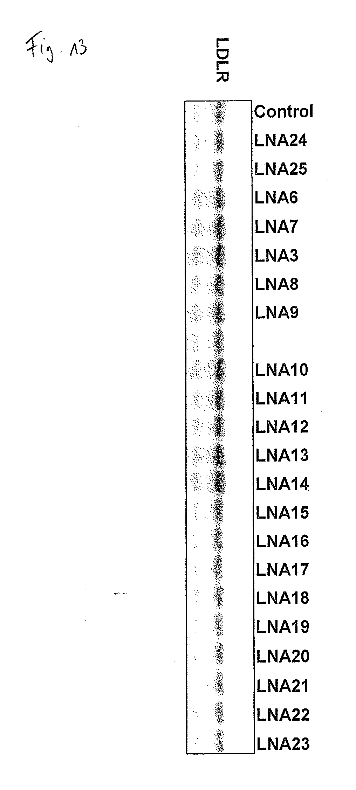

The LDLR expression level of the cells transfected with each polynucleotide analogue was evaluated, according to the methods described in "(2) Transfection of polynucleotide analogue" and "(3) Evaluation of LDLR expression level" of Example 1. The results obtained by Western blotting are shown in "Figure 13".

Compared to the cells without transfection of the polynucleotide analogue (control), cells transfected with each of LNAs 6 to 14 were found to be increased in the LDLR expression level. On the other hand, there was no significant increase in expression level, when the cells were transfected with each of LNAs 15 to 25.

Subsequently, it was examined whether LNAs 6 to 14 can inhibit binding of the ZFP family to ARE 1.

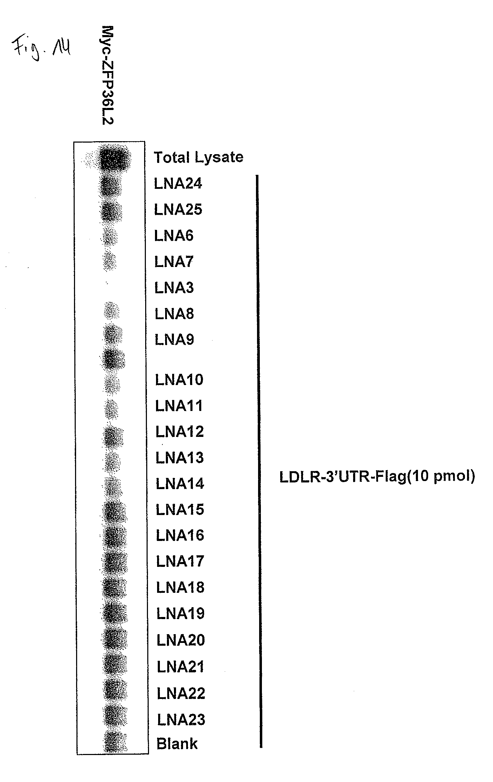

The flag-labeled LDLR 3'UTR was mixed with a cell extract protein extracted from 293T cells that were forced to express ZFP36L2 fused with Myc protein (Myc-tagged-ZFP36L2), and each of LNA 3 and LNAs 6 to 25 was added thereto for reaction, according to the method described in Example 3. The sample eluted after immunoprecipitation was subjected to Western blotting, by using an anti-Myc antibody. Results obtained by the Western blotting are shown in "Figure 14".

The sample obtained by mixing a cell extract protein containing Myc-tagged-ZFP36L2 with LDLR 3'UTR-flag and immunoprecipitating the product with an anti-flag antibody ("Blank" in the Figure) gave a large amount of ZFP36L2 detected. In contrast, samples obtained in reaction of a cell extract protein with LDLR 3'UTR-flag in the presence of LNAs 6 to 14, which showed an effectiveness of accelerating LDLR expression level, gave a distinctively lowered detection signal of ZFP36L2. The results confirm that LNAs 6 to 14 inhibit binding of ZFP36L2 to ARE1.

The fact that it was possible to regulate LDLR expression positively by using a polynucleotide analogue having a nucleotide sequence complementary to a particular ARE on LDLR mRNA in Examples 1 to 7 indicates that it would be possible to regulate gene expression of various genes in a similar manner, by modifying the nucleotide sequence of the polynucleotide or the like according to the present invention properly according to the target gene or the target cis element. It would also be possible to regulate LDLR expression not only positively but also negatively, by designing a polynucleotide or the like that can bind to an ARE other than the AREs 1 to 3 studied here or an cis element other than ARE and inhibiting the function of the cis element-binding factor other than ZFP36L1 and ZFP36L2. Therefore, it would be possible to regulate expression of various genes positively and negatively, by the gene expression regulation method according to the present invention.

### Brief Description of Drawings

Figure 1 is a schematic chart explaining the mechanism of regulating the stability of mRNA and translation amount thereof into the protein by a cis element and a cis element-binding factor.
Figure 2 is a schematic chart explaining an embodiment of the polynucleotide and the polynucleotide analogue according to the present invention.
Figure 3 is a chart showing the binding structures of DNA (A), PNA (B) and Morpholino (C).
Figure 4 is a schematic chart showing the locations of three AREs present in 3'UTR of LDLR mRNA.
Figure 5 is a chart showing the nucleotide sequences of the polynucleotide analogue LNAs 1 to 3 prepared in Example 1, which were designed by using ARE1 as target cis element, and the partial nucleotide sequence of the LDLR mRNA region containing ARE1.
Figure 6 is a chart showing the results obtained by evaluation of the expression level of LDLR by Western blotting in Example 1, wherein the top photo in Figure (A) shows the band of LDLR detected while the bottom photo shows the band of β-actin detected, and Figure (B) shows the quantitative concentrations of the detected bands, as expressed as relative values.
Figure 7 is a chart showing the results obtained by evaluation of the binding potential of ZFP36L1 and ZFP36L2 to LDLR mRNA 3'UTR and the binding-inhibiting potential of LNAs 1 to 3 by Western blotting in Example 3.
Figure 8 is a chart showing the results of Western blotting in Example 4 that was obtained by evaluation of the change in LDLR expression level when expression of ZFP36L1 and ZFP36L2 is inhibited by using RNAi's, wherein the top photo in Figure (A) shows the band of LDLR detected, while the bottom photo shows the band of β-actin detected, and Figure (B) shows the quantitative concentrations of the detected bands, as expressed as relative values.
Figure 9 is a schematic chart explaining the mechanism of enhancement of LDLR expression by the polynucleotide or the like according to the present invention.
Figure 10 is a chart showing the results obtained by evaluation of LDLR expression level by Western blotting in Example 5, wherein the top band in Figure (A) represents the band of LDLR detected, while the bottom band represents the band of β-actin detected.
Figure 11 is a schematic chart illustrating the structure of the mRNA baits prepared by deletion of AREs 1 to 3 in Example 6.
Figure 12 is a chart showing the results obtained by evaluation of the binding potential of ZFP36L1 and ZFP36L2 to AREs 1 to 3 by Western blotting in Example 6.
Figure 13 is a chart showing the results obtained by evaluation of LDLR expression level by Western blotting in Example 7.
Figure 14 is a chart showing the results obtained by evaluation of the binding-inhibiting potential of LNAs 6 to 25 to ZFP36L2 by Western blotting in Example 7.

## Claims

1. A polynucleotide or polynucleotide analogue, comprising a nucleotide sequence complementary to at least part of a cis element of mRNA and a nucleotide sequence complementary to part of the 5'- and/or 3'- untranslated region of the cis element, wherein the polynucleotide or polynucleotide analogue inhibits binding of the cis element-binding factor to the cis element by binding to at least part of the cis element specifically, based on the sequence specificity of the nucleotide sequence complementary to the untranslated region.

2. The polynucleotide or polynucleotide analogue according to Claim 1, wherein the polynucleotide or polynucleotide analogue stabilizes the mRNA selectively and accelerates translation thereof into the gene product or destabilizes the mRNA and inhibits translation thereof into the gene product, by inhibiting binding of the cis element-binding factor to the cis-element

3. The polynucleotide or polynucleotide analogue according to Claim 2, wherein the polynucleotide or polynucleotide analogue inhibits binding of an AU-rich element binding factor to an AU-rich element.

4. The polynucleotide or polynucleotide analogue according to Claim 3, wherein the mRNA is LDLR mRNA and the polynucleotide or polynucleotide analogue inhibits binding of ZFP36L1 and/or ZFP36L2 to the AU-rich element.

5. The polynucleotide or polynucleotide analogue according to Claim 3 or 4, wherein the mRNA is LDLR mRNA and the polynucleotide or polynucleotide analogue inhibits binding of the cis element-binding factor to ARE1 (2790 to 2797 bases from the 5'- terminal in the nucleotide sequence of LDLR mRNA represented by SEQ ID No. 4).

6. The polynucleotide or polynucleotide analogue according to Claim 4 or 5, having a nucleotide sequence represented by any one of SEQ ID Nos. 1 to 3 and 6 to 14 or a nucleotide sequence substantially equivalent to the nucleotide sequence.

7. A LDLR expression enhancer, comprising the polynucleotide and/or polynucleotide analogue according to any one or more of Claims 4 to 6 or an expression vector that can express any one or more of the polynucleotides.

8. A pharmaceutical composition for prevention, alleviation, or treatment of hyperlipidemia or hyperlipidemia-related diseases, comprising the LDLR expression enhancer according to Claim 7 as the active ingredient.

9. The pharmaceutical composition according to Claim 8, wherein the hyperlipidemia-related disease is one or more disease selected from the group consisting of arteriosclerosis, hypertension, cerebral infarction, myocardial infarction, angina cordis, diabetes, adiposis, and cancer.

10. A pharmaceutical composition for prevention, alleviation, or treatment of Alzheimer's disease, comprising the LDLR expression enhancer according to Claim 7 as the active ingredient.

11. An expression vector, capable of expressing the polynucleotide according to Claim 6.

12. A gene product expression enhancer or inhibitor, comprising the polynucleotide and/or polynucleotide analogue according to Claim 1 or an expression vector capable of expressing the polynucleotide.

13. A pharmaceutical composition, comprising the gene product expression enhancer or inhibitor according to Claim 12 as the active ingredient.

14. Use of the polynucleotide or polynucleotide analogue according to Claim 1 or the expression vector capable of expressing the polynucleotide, in production of a gene product expression enhancer or inhibitor.

15. An expression vector, capable of expressing the polynucleotide according to Claim 1.

16. The polynucleotide analogue according to Claim 1, comprising LNA.

17. A gene expression regulation method, comprising regulating expression of a target gene product by inhibiting binding of a cis element-binding factor to a cis element, by using a polynucleotide and/or polynucleotide analogue having a nucleotide sequence complementary to at least part of the cis element of mRNA and a nucleotide sequence complementary to part of the 5'- and/or 3'-untranslated region of the cis element.
